# EUROPEAN PATENT SPECIFICATION

(11) **EP 2 621 334 B1**
(45) Date of publication and mention of the grant of the patent: **11.09.2019**
(21) Application number: 11770606.9
(22) Date of filing: 30.09.2011
(51) Int. Cl.: A61B 5/00

(54) **DETECTING, QUANTIFYING, AND/OR CLASSIFYING SEIZURES USING MULTIMODAL DATA**
NACHWEIS, QUANTIFIZIERUNG UND/ODER KLASSIFIZIERUNG VON ANFÄLLEN ANHAND VON MULTIMODALEN DATEN
DÉTECTION, QUANTIFICATION ET/OU CLASSIFICATION DE CRISES ÉPILEPTIQUES AU MOYEN DE DONNÉES MULTIMODALES

(30) Priority: 29.04.2011 US 201113098262; 01.10.2010 US 896525
(43) Date of publication of application: 07.08.2013
(73) Proprietor: Flint Hills Scientific, L.L.C., Lawrence, KS 66044 (US)
(72) Inventor: OSORIO, Ivan, Leawood KS 66209 (US)
(74) Representative: Grünecker Patent- und Rechtsanwälte PartG mbB
(86) International application number: PCT/US2011/054287
(87) International publication number: WO 2012/044970

(56) References cited:
- WO-A1-2006/134359
- WO-A1-2011/126931
- US-A- 5 928 272
- US-A- 5 995 868
- US-A1- 2004 267 152
- US-A1- 2008 319 281
- US-A1- 2009 137 921

## Description

### FIELD OF THE INVENTION

This disclosure relates to medical device systems and methods capable of detecting and, in some embodiments, treating an occurring or impending seizure using multimodal body data.

### DESCRIPTION OF THE RELATED ART

Of the approximately 60 million people worldwide affected with epilepsy, roughly 23 million people suffer from epilepsy resistant to multiple medications. In the USA alone, the annual cost of epilepsy care is USD 12 billion (in 1995 dollars), most of which is attributable to subjects with pharmaco-resistant seizures. Pharmaco-resistant seizures are associated with an increase mortality and morbidity (e.g., compared to the general population and to epileptics whose seizures are controlled by medications) and with markedly degraded quality of life for patients. Seizures may impair motor control, responsiveness to a wide class of stimuli, and other cognitive functions. The sudden onset of a patient's impairment of motor control, responsiveness, and other cognitive functions precludes the performance of necessary and even simple daily life tasks such as driving a vehicle, cooking, or operating machinery, as well as more complex tasks such as acquiring knowledge and socializing.

Therapies using electrical currents or fields to provide a therapy to a patient (electrotherapy) are beneficial for certain neurological disorders, such as epilepsy. Implantable medical devices have been effectively used to deliver therapeutic electrical stimulation to various portions of the human body (e.g., the vagus nerve) for treating epilepsy. As used herein, "stimulation," "neurostimulation," "stimulation signal," "therapeutic signal," or "neurostimulation signal" refers to the direct or indirect application of an electrical, mechanical, magnetic, electro-magnetic, photonic, acoustic, cognitive, and/or chemical signal to an organ or a neural structure in the patient's body. The signal is an exogenous signal that is distinct from the endogenous electro-chemical activity inherent to the patient's body and also from that found in the environment. In other words, the stimulation signal (whether electrical, mechanical, magnetic, electro-magnetic, photonic, acoustic, cognitive, and/or chemical in nature) applied to a cranial nerve or to other nervous tissue structure in the present disclosure is a signal applied from a medical device, e.g., a neurostimulator.

A "therapeutic signal" refers to a stimulation signal delivered to a patient's body with the intent of treating a medical condition through a suppressing (e.g., blocking) or modulating effect to neural tissue. The effect of a stimulation signal on neuronal activity may be suppressing or modulating; however, for simplicity, the terms "stimulating", suppressing, and modulating, and variants thereof, are sometimes used interchangeably herein. In general, however, the delivery of an exogenous signal itself refers to "stimulation" of an organ or a neural structure, while the effects of that signal, if any, on the electrical activity of the neural structure are properly referred to as suppression or modulation.

Depending upon myriad factors such as the history (recent and distant) of a patient's brain activity (e.g., electro-chemical, mental, emotional), stimulation parameters and time of day, to name a few, the effects of stimulation upon the neural tissue may be excitatory or inhibitory, facilitatory or disfacilitatory and may suppress, enhance, or leave unaltered neuronal activity. For example, the suppressing effect of a stimulation signal on neural tissue would manifest as the blockage of abnormal activity (e.g., epileptic seizures) see Osorio et al., Ann Neurol 2005; Osorio & Frei IJNS 2009) The mechanisms thorough which this suppressing effect takes place are described in the foregoing articles. Suppression of abnormal neural activity is generally a threshold or suprathreshold process and the temporal scale over which it occurs is usually in the order of tens or hundreds of milliseconds. Modulation of abnormal or undesirable neural activity is typically a "sub-threshold" process in the spatio-temporal domain that may summate and result under certain conditions, in threshold or suprathreshold neural events. The temporal scale of modulation is usually longer than that of suppression, encompassing seconds to hours, even months. In addition to inhibition or dysfacilitation, modification of neural activity (e.g., wave annihilation) may be exerted through collision with identical, similar or dissimilar waves, a concept borrowed from wave mechanics, or through phase resetting (Winfree).

In some cases, electrotherapy may be provided by implanting an electrical device, *e.g*., an implantable medical device (IMD), inside a patient's body for stimulation of a nervous tissue, such as a cranial nerve. Generally, electrotherapy signals that suppress or modulate neural activity are delivered by the IMD via one or more leads. When applicable, the leads generally terminate at their distal ends in one or more electrodes, and the electrodes, in turn, are coupled to a target tissue in the patient's body. For example, a number of electrodes may be attached to various points of a nerve or other tissue inside a human body for delivery of a neurostimulation signal.

While contingent (also referred to as "closed-loop," "active," or "feedback" stimulation; *i.e.,* electrotherapy applied in response to sensed information, such as heart rate) stimulation schemes have been proposed, non-contingent, programmed periodic stimulation is the prevailing modality. For example, vagus nerve stimulation for the treatment of epilepsy usually involves a series of grouped electrical pulses defined by an "on-time" (such as 30 sec.) and an "off-time" (such as 5 min.). This type of stimulation is also referred to as "open-loop," "passive," or "non-feedback" stimulation. Each sequence of pulses during an on-time may be referred to as a "pulse burst." The burst is followed by the off-time period in which no signals are applied to the nerve. During the on-time, electrical pulses of a defined electrical current (e.g., 0.5 - 3.5 milliamps) and pulse width (e.g., 0.25 - 1.0 milliseconds) are delivered at a defined frequency (e.g., 20 - 30 Hz) for a certain duration (e.g., 10 - 60 seconds). The on-time and off-time parameters together define a duty cycle, which is the ratio of the on-time to the sum of the on-time and off-time, and which describes the fraction of time that the electrical signal is applied to the nerve.

In VNS, the on-time and off-time may be programmed to define an intermittent pattern in which a repeating series of electrical pulse bursts are generated and applied to a cranial nerve such as the vagus nerve. The off-time is provided to minimize adverse effects and conserve power. If the off-time is set at zero, the electrical signal in conventional VNS may provide continuous stimulation to the vagus nerve. Alternatively, the off time may be as long as one day or more, in which case the pulse bursts are provided only once per day or at even longer intervals. Typically, however, the ratio of "off-time" to "on-time" may range from about 0.5 to about 10.

In addition to the on-time and off-time, the other parameters defining the electrical signal in VNS may be programmed over a range of values. The pulse width for the pulses in a pulse burst of conventional VNS may be set to a value not greater than about 1 msec, such as about 250-500 µsec, and the number of pulses in a pulse burst is typically set by programming a frequency in a range of about 20-300 Hz (i.e., 20 pulses per second to 300 pulses per second). A non-uniform frequency may also be used. Frequency may be altered during a pulse burst by either a frequency sweep from a low frequency to a high frequency, or vice versa. Alternatively, the timing between adjacent individual signals within a burst may be randomly changed such that two adjacent signals may be generated at any frequency within a range of frequencies.

Although neurostimulation has proven effective in the treatment of a number of medical conditions, it would be desirable to further enhance and optimize neurostimulation-based therapy for this purpose. For example, it may be desirable to detect an occurring or impending seizure. Such detection may be useful in triggering a therapy, monitoring the course of a patient's disease, or the progress of his or her treatment thereof. Alternatively or in addition, such detection may be useful in issuing a warning of an impending or on-going seizure. Such a warning may, for example, minimize the risk of injury or death. Said warning may be perceived by the patient, a physician, a caregiver, or a suitably programmed computer and allow that person or computer program to take action intended to reduce the likelihood, duration, or severity of the seizure or impending seizure, or to facilitate further medical treatment or intervention for the patient. In particular, detection of an occurring or impending seizure enables the use of contingent neurostimulation. The state of the art does not provide an efficient and effective means for performing such detection and/or warning. Conventional V S stimulation as described above does not detect occurring or impending seizures.

Closed-loop neurostimulation therapies for treating epilepsy have been proposed in which stimulation is triggered based upon factors including EEG activity (see, e.g., US 5,995,868 and US 7,280,867) as well as cardiac-based activity (see., e.g., US 6,961,618 and US 5,928,272). EEG- or ECoG-based approaches involving recording of neural electrical activity at any spatio-temporal scale involve determination of one or more parameters from brain electrical activity that indicate a seizure. Such approaches have met with limited success and have a number of drawbacks, including highly invasive and technically demanding and costly surgery for implanted systems. Approaches that do not invade the brain have marked limitations due mainly to the extremely low/unreliable S/N, and poor patient compliance with, e.g., the patient wearing electrodes on the scalp for extended periods.

US 2008/0319281 discloses a portable device for detecting a medical condition, such as an epileptic seizure. The device is implemented on a wrist band, possibly together with a watch, or in a helmet. The device may use heart rate detection to identify characteristic patterns associated with epileptic seizure. The device optionally combines more than one measurement to eliminate false positives. In the case of epileptic seizures, heart rate related measurements may be combined with body motion related measurements to ensure greater accuracy.

### SUMMARY OF THE INVENTION

According to the present invention, it is provided a medical device system for detecting an epileptic seizure based upon a patient's cardiac signal and body movement as defined in claims 1 and 7.

Preferred embodiments of the invention are defined in the dependent claims.

The following description is for illustrative purpose only.

### BRIEF DESCRIPTION OF THE DRAWINGS

The disclosure may be understood by reference to the following description taken in conjunction with the accompanying drawings, in which like reference numerals identify like elements, and in which:

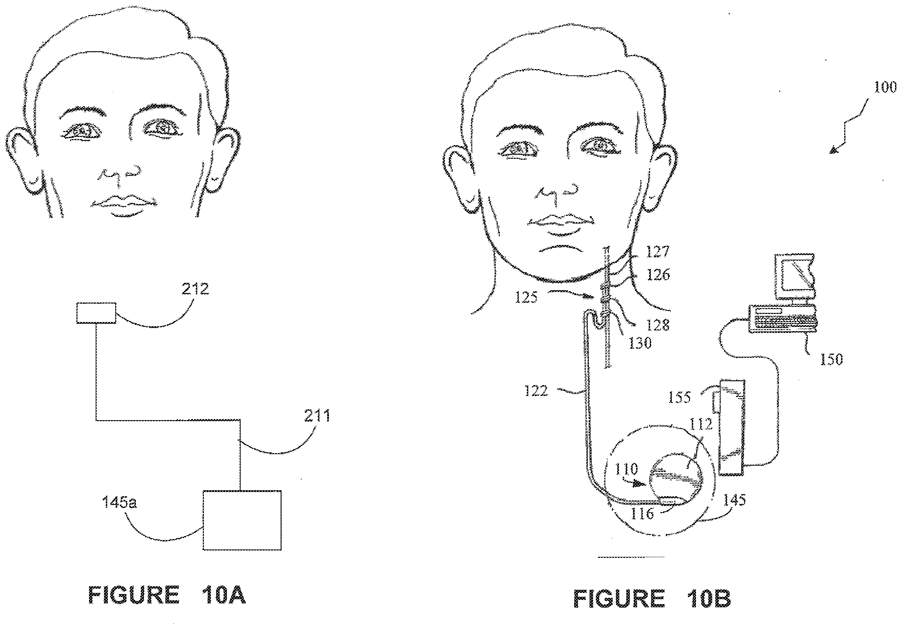

Figure 10 provides stylized diagrams of medical devices. Figure 10A shows an external device in communication with a sensor. Figure 10B shows an implanted device providing a therapeutic signal to a structure of the patient's body, each in accordance with one illustrative embodiment of the present disclosure;
Figure 1 shows the time of appearance (relative to clinical onset, dashed vertical line) and direction of deviations from reference activity of a plurality of body signals for multiple seizure types, specifically, absence seizures, tonic-clonic seizures, and simple or complex partial seizures;
Figure 2 shows time courses (relative to clinical onset, dashed vertical line) of activity of a plurality of body signals for tonic-clonic seizures;
Figure 3 shows time courses (relative to clinical onset, dashed vertical line) of activity of a plurality of body signals for partial (simple or complex) seizures;
Figure 4 shows time courses (relative to clinical onset, dashed vertical line) of activity of a plurality of body signals for idiopathic absence seizures;
Figure 5 shows (A) an exemplary two-dimensional plot of a trajectory of epileptic movements, (B) an exemplary three-dimensional plot of epileptic movements, and (C) an additional exemplary three-dimensional plot of epileptic movements;
Figure 6 shows three two-dimensional, temporally cumulative plots of discrete movements during the clonic phase of a primarily or secondarily generalized tonic-clonic seizure;
Figure 7 shows a flowchart of an implementation of a method according to one embodiment of the present disclosure;
Figure 8 shows a flowchart of an implementation of a method according to one embodiment of the present disclosure;
Figure 9 shows a flowchart of an implementation of a method according to one embodiment of the present disclosure;
Figure 11 provides a block diagram of a medical device system that includes a medical device and an external unit, in accordance with one illustrative embodiment of the present disclosure;
Figure 12A provides a block diagram of an autonomic signal module of a medical device, in accordance with one illustrative embodiment of the present disclosure;
Figure 12B provides a block diagram of a neurologic signal module of a medical device, in accordance with one illustrative embodiment of the present disclosure; and
Figure 12C provides a block diagram of a detection module of a medical device, in accordance with one illustrative embodiment of the present disclosure.

While the disclosure is susceptible to various modifications and alternative forms, specific embodiments thereof have been shown by way of example in the drawings and are herein described in detail. It should be understood, however, that the description herein of specific embodiments is not intended to limit the disclosure to the particular forms disclosed, but on the contrary, the intention is to cover all modifications, equivalents, and alternatives falling within the appended claims.

### DESCRIPTION OF ILLUSTRATIVE EMBODIMENTS

Illustrative embodiments of the disclosure are described herein. In the interest of clarity, not all features of an actual implementation are described in this specification. In the development of any such actual embodiment, numerous implementation-specific decisions must be made to achieve the design-specific goals, which will vary from one implementation to another. It will be appreciated that such a development effort, while possibly complex and time-consuming, would nevertheless be a routine undertaking for persons of ordinary skill in the art having the benefit of this disclosure.

This document does not intend to distinguish between components that differ in name but not function. In the following discussion and in the claims, the terms "including" and "includes" are used in an open-ended fashion, and thus should be interpreted to mean "including, but not limited to." Also, the term "couple" or "couples" is intended to mean either a direct or an indirect electrical connection. "Direct contact," "direct attachment," or providing a "direct coupling" indicates that a surface of a first element contacts the surface of a second element with no substantial attenuating medium there between. The presence of small quantities of substances, such as bodily fluids, that do not substantially attenuate electrical connections does not vitiate direct contact. The word "or" is used in the inclusive sense (*i.e*., "and/or") unless a specific use to the contrary is explicitly stated.

The term "electrode" or "electrodes" described herein may refer to one or more stimulation electrodes (i.e., electrodes for delivering a therapeutic signal generated by an IMD to a tissue), sensing electrodes (i.e., electrodes for sensing a physiological indication of a state of a patient's body), and/or electrodes that are capable of delivering a therapeutic signal, as well as performing a sensing function.

Identification of changes in brain state (whether physiologic or pathologic) has traditionally been accomplished through analysis of electrical brain signals and behavioral observation. Continuous (e.g., round-the-clock) automated monitoring of changes in brain state imposes certain limitations on the utilization of these traditional methods, due to the difficulties inherent to automated ambulatory video, the large amount of data produced per unit time, and the excessive demands on human and technical resources required to maintain an acceptable signal/noise for electrical signals recorded from the scalp. Additionally, scalp signals have poor temporo-spatial resolution, a characteristic which results in both low sensitivity and specificity of state-of-brain detection changes.

Implanted sensors or electrodes beneath the scalp but above the outer skull table or intra-cranial (epidural, subdural or depth) have been used to overcome the limitations of scalp recordings. However, the quality of data is limited; there are risks (e.g., infection, bleeding, brain damage) associated with these devices; and in addition, at this time, there are at most about 300 neurosurgeons in the United States capable of implanting intracranial electrodes, far too few to perform such implantation for the roughly 900,000 pharmaco-resistant epileptics in the United States.

While electrical brain signals and behavioral observation may provide information for classification of brain states, this task can be accomplished more efficiently, more precisely, and/or more cost-effectively through monitoring of other biological signals such those generated by the heart, muscle, skin, eyes, tympanic membrane temperature, and body posture/movement, since they may not require surgery, or if surgery is required for implantation, the procedures are much shorter, simpler, and cheaper that those required for recording of brain signals and there is no shortage of human resources.

Certain highly valuable neurological signals (e.g., cognitive) for detection, quantification, and classification of state changes may obtained non-invasively and can be used in this disclosure.

These multi-modal (e.g., autonomic, neurologic, etc.) signals can be used individually or in combination to monitor continuously the brain and generate a state-of the-system/organ report, in real-time for the detection, quantification, classification, validation, control and logging of physiologic or pathologic state changes. This approach takes advantage of the inherent and finely tuned dynamical coupling among these systems. For instance, changes in brain state/activity may result in changes in heart activity, muscle activity, and skin properties.

Herein, Applicant describes a method, systems, and devices that may: a) detect in real-time pre-specified changes in brain state; b) quantify their duration, intensity, and time of occurrence; c) classify their type (e.g., epileptic vs. non-epileptic seizures; primarily vs. secondarily generalized seizures; generalized vs. partial seizures; complex vs., simple partial seizures; d) use as a basis for warning and control/therapy, and/or e) save this information to memory for future retrieval for optimization of detection, quantification and classification of state changes and assessment and optimization of therapeutic (e.g., control) efficacy. Non-epileptic movements in this disclosure refer to those resembling movements seen during tonic-clonic seizures but which are not caused by those seizures.

Herein, "multimodal" refers to epileptic event detection based on more than one endogenous mode or type of signal. The multimodal epileptic event detection disclosed herein provides a comprehensive, cost-effective, valuable alternative to systems of epileptic event detection exclusively based on brain electrical signals such as EEG. To date, no multimodal systems have been developed or commercialized. Multimodal epileptic event detection may make use of signals or markers of autonomic, neurologic, endocrine, metabolic, gastro-intestinal, and/or dermal origin and of tissue/organ stress, such as those presented in Table 1.

Multimodal detection of state changes takes advantage of the fact that certain brain structures directly or indirectly influence autonomic, endocrine, gastro-intestinal, dermal and metabolic functions and that certain abnormal states (e.g. seizures) stress the body tissues and result in the elevation of certain compounds or molecules (e.g., stress markers) that may be used to detect and verify the occurrence of said abnormal state.

It has been established that seizures in humans originating from or spreading to central autonomic structures induce changes in heart rate, among other cardio-vascular indices or features. It should be stated that seizure-induced heart rate increases (which are far more frequent than heart rate decreases) are not primarily the result of increased motor activity or of metabolic changes, but are instead a neurogenic phenomenon. In the present disclosure, a highly robust, efficient and reliable system is provided for detecting, quantifying and/or classifying epileptic seizures based upon multi-modal signals and, if desired, using this information to provide warnings, therapies and optimization of all of these tasks. Systems of the present disclosure are suitable for commercial, long-term implants or external devices and provide reliable and accurate indications of seizure events for a wide variety of epilepsy patients. Various multimodal signals that may be used in the disclosure are set forth in the following table:

### Multimodal Signals (Table 1)

### Autonomic

Cardiac: EKG, PKG, Echocardiography, Apexcardiography (ApKG), Intra-cardiac pressure, Cardiac blood flow, cardiac thermography; from which can be derived, e.g., heart rate (HR), change of HR, rate of change of HR, heart rhythm, changes in heart rhythm, heart rate variability (HRV), change of HRV, rate of change of HRV, HRV vs. HR. Also, heart morphology (e.g., size) blood pressure (arterial and venous), heart sounds, , heartbeat wave morphology, heartbeat complex morphology, and magnitude and shape of thoracic wall deflection.

Vascular: Arterial Pressure, Arterial and venous blood wave pressure morphology; Arterial and venous blood flow velocity and degree of turbulence, arterial and venous blood flow sounds, arterial and venous temperature
Respiratory: Frequency, tidal volume, minute volume, respiratory wave morphology, respiratory sounds, end-tidal CO2, Intercostal EMG, Diaphragmatic EMG, chest wall and abdominal wall motion, from which can be derived, e.g.,, respiration rate (RR), change of RR, rate of change of RR, respiratory rhythm, morphology of breaths. Also, arterial gas concentrations, including oxygen saturation, as well as blood pH can be considered respiratory signals.

Dermal: Skin resistance, skin temperature, skin blood flow, sweat gland activity
Concentrations of catecholamines (and their metabolites) and acetylcholine or acetylcholinesterase activity in blood, saliva and other body fluids concentrations and its rate of change.

### Neurologic

Cognitive/behavioral: Level of consciousness, attention, reaction time, memory, visuo-spatial, language, reasoning, judgment, mathematical calculations, auditory and/or visual discrimination
Kinetic: Direction, speed/acceleration, trajectory (ID to 3D), pattern, and quality of movements, force of contraction, body posture, body orientation/position, body part orientation/position in reference to each other and to imaginary axes, muscle tone, agonist-to-antagonist muscle tone relation, from which can be derived, e.g., information about gait, posture, accessory movements, falls
Vocalizations: Formed, unformed
EEG/ECoG, Evoked potentials, field potentials, single unit activity
Endocrine: Prolactin, luteinizing hormone, follicle stimulation hormone, growth hormone, ACTH, cortisol, vasopressin, beta-endorphin, beta, lipotropin-, corticotropin-releasing factor (CRF)
Stress Markers: CK, troponin, reactive oxygen and nitrogen species including but not limited to iso- and neuro-prostanes and nitrite/nitrate ratio, gluthatione, gluthatione disulfide and gluthatione peroxidase activity, citrulline, protein carbonyls, thiobarbituric acid, the heat shock protein family, catecholamines, lactic acid, N-acetylaspartate, and metabolites of any of the foregoing.

Metabolic: arterial pH and gases, lactate/pyruvate ratio, electrolytes, glucose

In one embodiment, the present disclosure relates to systems and methods for detecting an epileptic event based upon an autonomic signal (*e.g*., a cardiac signal) and a neurologic signal (*e.g*.*,*, a kinetic signal) of a patient, comprising providing an autonomic signal indicative of the patient's autonomic activity; providing a neurologic signal indicative of the patient's neurological activity; detecting an epileptic event based upon the autonomic signal and the neurologic signal.

"Epileptic event" refers to a seizure, a period of increased likelihood of a seizure, a pre-ictal period, or a post-ictal period, among others.

Any autonomic signal indicative of the patient's autonomic activity can be used in the method. In one embodiment, the autonomic signal is selected from the group consisting of a cardiac signal, a respiratory signal, a skin resistivity signal, an eye signal, a blood signal, and two or more thereof. The autonomic signal can be provided by an electrocardiogram (EKG) device, a pupillometer, a face or body temperature monitor, a skin resistance monitor, a sound sensor, a pressure sensor, a blood gas sensor, among others, or two or more thereof.

Any neurologic signal indicative of the patient's neurological activity can be used in the method. In one embodiment, the neurologic signal is selected from the group consisting of a brain signal, a kinetic signal, and two or more thereof. The neurologic signal can be provided by an electroencephalography (EEG) device, an electrocorticography (ECoG) device, an accelerometer, an inclinometer, an actigraph, a responsiveness testing device or system, among others, or two or more thereof.

An epileptic event can be detected based upon the autonomic signal and the neurologic signal.

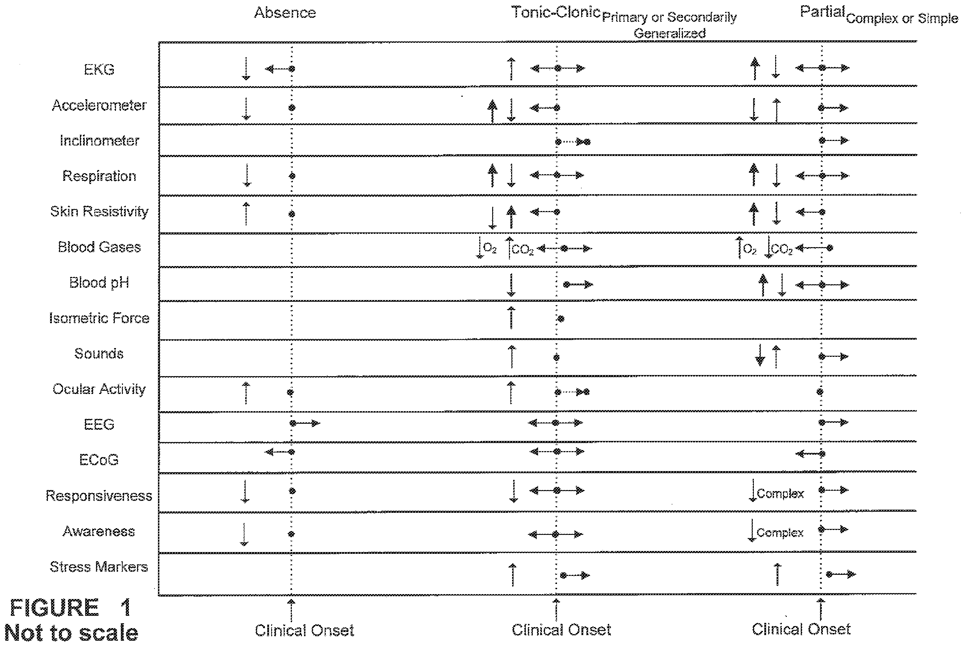

In one embodiment, when the autonomic signal is a cardiac signal, the detection can be partially based on the observation that some seizure types are associated with a change (e.g., increase) in heart rate compared to a reference heart rate value range, such as a range of measures of central tendency of heart rate over a short or relatively long time window. Some other seizure types are associated with a decrease in heart rate above a reference heart rate value (see for example, Figure 1).

Generally, when the term "reference value" is used herein without further qualification, it refers to a value derived from an interictal period. Reference values or ranges thereof for any of the autonomic, neurologic, endocrine, metabolic or stress marker features are day of time (e.g., circadian) and state (e.g., resting wakefulness) dependent and thus non-stationary. Although reference values for a certain feature in a certain state or time are most directly comparable to corresponding signals in the same state or time, they may be comparable to corresponding signals from other states, times, or both.

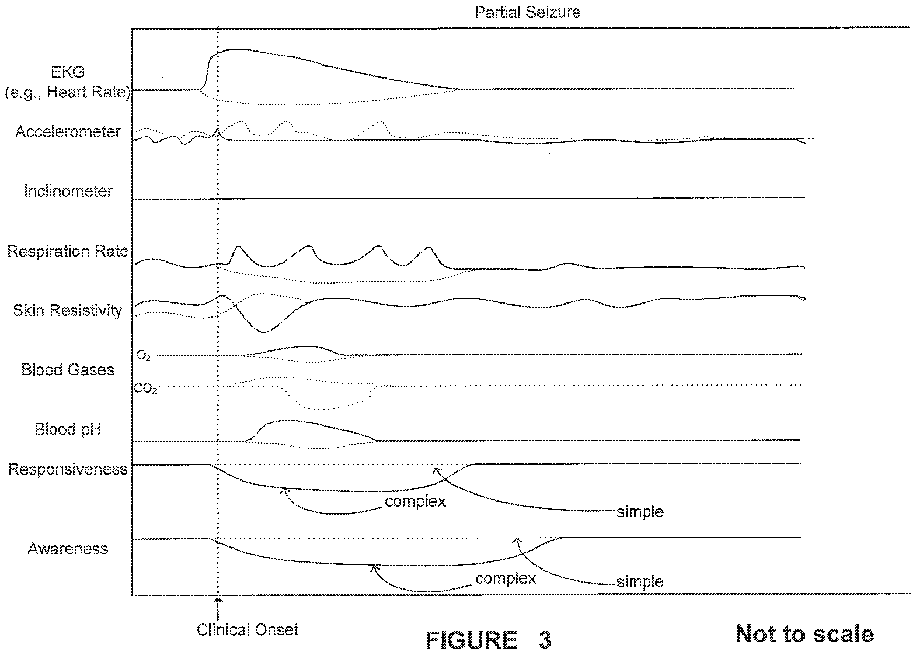

As used in Figures 1-4, clinical onset refers to the earlier of either a) when a patient notices a first seizure symptom, or b) when an expert observer (or a person familiar with the patient's seizures) observes a first change indicative of the seizure. It must be underscored that while the most apparent change may be the "first" to be noticed by the patient or seen by the observer, this change may have been preceded by other (unnoticed or unobserved) changes, and that the "first change" defining the seizure onset may not be the first change actually occurring and associated with the seizure. Only one of several indicia or signs of a seizure may be clinically recognizable, and the clinical onset time is thus given or determined by this clinically recognizable sign or symptom. This does not preclude other clinical symptoms having occurred prior to the clinically recognizable sign or symptom. This is illustrated, for example, in Figure 3, in which the onset of impaired responsiveness precedes, by a few seconds, the clinical onset, and where the EKG and respiratory changes are also shown as occurring before clinical onset.

Figure 1 shows the time of appearance (relative to clinical onset, dashed vertical line) and direction of deviations from interictal reference activity, of a plurality of body signals for four seizure types:, specifically, absence seizures, generalized tonic-clonic seizures (whether primarily or secondarily generalized), and simple or complex partial seizures. The horizontal arrows show the times of appearance of the symptom change in reference to clinical onset as defined in the present application. A dot without horizontal arrows indicates that the most important aspect of the signal change occurs at clinical onset. This does not exclude the possibility that this change may reappear or change direction at some later time. Upward vertical arrows indicate an increase in the value of the signal while downwards arrows indicate a decrease in value. Arrow length does not reflect a scale or magnitude of the change. When multiple deviations are shown, the larger, thicker arrow is the one most commonly seen over general patient populations. Of course, the skilled epileptologist is aware that some patients will show one or more variations from the typical cases shown in Figure 1.

To facilitate understanding, certain important details about certain body signals, their onset, and temporal evolution in reference to clinical onset (dashed lines) have been omitted from Figures 1-4. These figures should be viewed only as illustrative of the changes in body signals that occur with the various seizure classes.

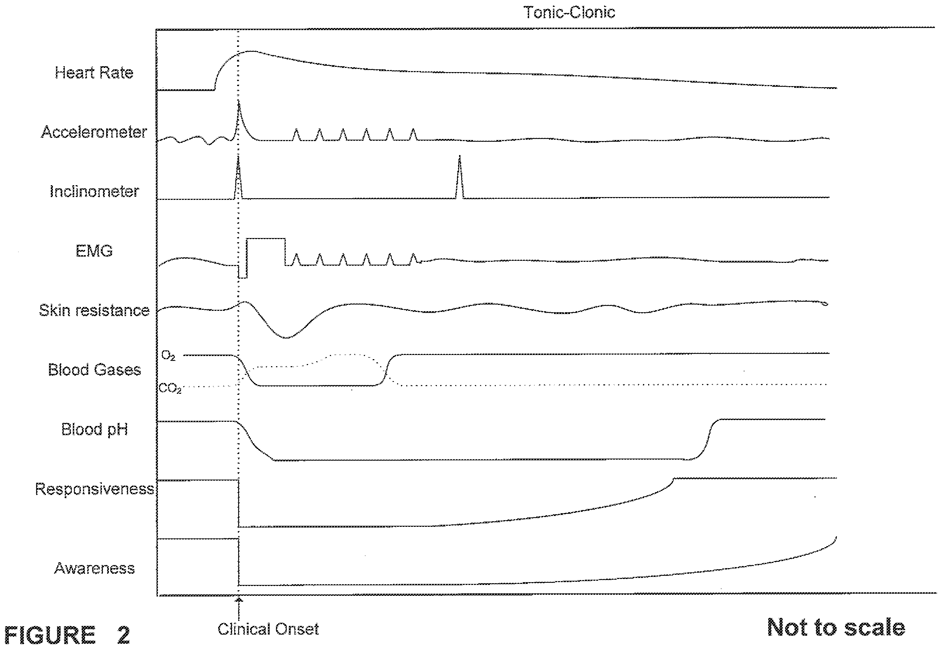

For example, tonic-clonic seizures are often correlated with an increase in heart rate beginning at about seizure onset (see for example, Figure 2).

For another example, partial seizures are often correlated with an increase in heart rate beginning before, at, or shortly after electrographic seizure onset. The increase is less than that associated with tonic-clonic seizures (see for example, Figure 3).

In another embodiment, when the autonomic signal is a respiratory signal, the detection can be partially based on the observation that some seizure types are associated with a deviation of the respiration rate from a reference respiration rate value range (see for example, Figure 1).

For example, partial seizures are often correlated with increases in respiration rate (see for example, Figure 3).

In one embodiment, when the autonomic signal is a skin resistivity signal, the detection can be partially based on the observation that some seizure types are associated with a deviation of skin or body temperature from an interictal reference skin or body temperature value range (see for example, Figure 1).

For example, certain partial seizures are associated with a decrease in skin resistivity (see for example, Figure 3) and tonic-clonic seizures with an increase in body temperature.

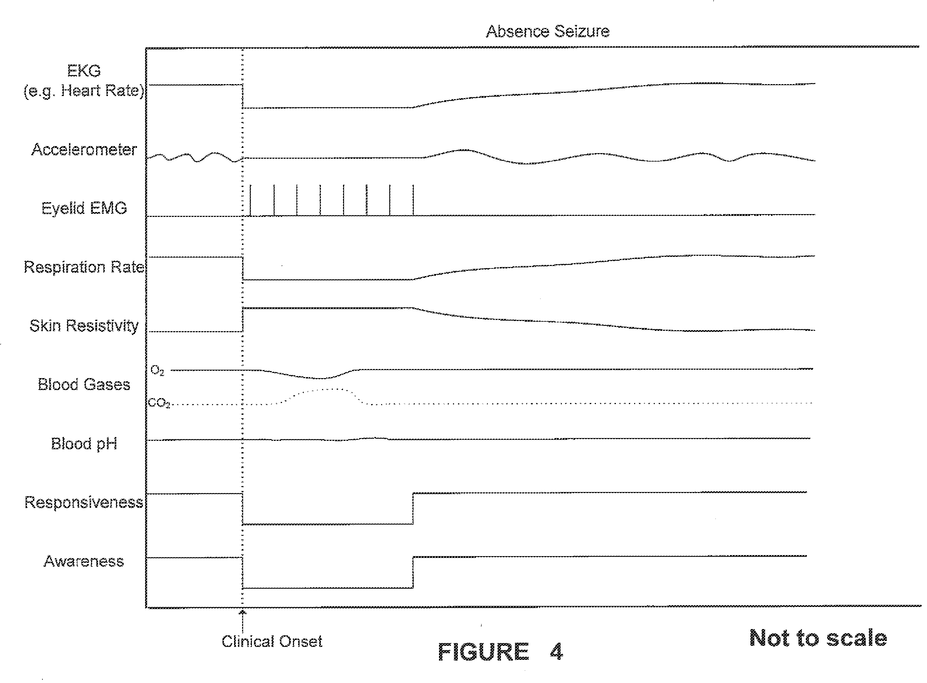

In still another embodiment, when the neurologic signal is an eye signal, the detection can be partially based on the observation that some seizure types are associated with eye position changes (e.g., forced binocular deviation to the right) or the occurrence of abnormal eye movements (e.g., horizontal nystagmus) or both) (see for example, Figure 1). For example, absence seizures are associated with quasiperiodic blinking (see for example, Figure 4).

The rate, amplitude and pattern of eyelid blinking may provide information about level of consciousness (*e.g*., awake vs. asleep or unresponsive) of a patient and during wakefulness. These parameters may allow for differentiation of normal vs. abnormal wakefulness states, *e.g*., abnormal wakeful state during complex partial and/or absence seizures, the state following termination of complex partial and/or absence seizures, and/or the termination of generalized tonic clonic seizures. Parameters such as blinking rate, amplitude and inter-blinking interval (from which distinctive patterns may be discerned) may be used for detection and quantification of seizures as well as for classification purposes through comparisons with the non-seizure interictal state. Blinking activity, which is a form of kinetic activity, may be recorded using device(s) (*e.g*., electrodes) placed over or under the skin overlaying the supra- or infraorbital regions or with optical devices.

In one embodiment, when the autonomic signal is a blood signal, the detection can be partially based on the observation that some seizure types are associated with an increase in stress markers (e.g. catecholamines, cortisol, and metabolites thereof) relative to a reference level of the stress marker.

Should stress markers reach a prespecified reference value (which may be different than that used for detection of state change purposes), the patient's total antioxidant capacity and lipid peroxidation intensity may be monitored to institute neuroprotective measures, such as increasing total antioxidant capacity. Neuronal hyper-excitability which occurs in seizures may lead to excessive production of free radicals and eventually to neuronal injury.

Alternatively or in addition, the blood signal can be a blood gas (e.g., O₂ or CO₂) level or a blood pH level, and the detection can be partially based on the observation that some seizure types are associated with blood gas and/or pH levels outside of an interictal reference value range (see for example, Figure 1). One or more of the blood signals described above may give information regarding respiratory signals, and vice versa.

For example, tonic-clonic seizures are associated with a drop in arterial O₂ concentration, an increase in arterial CO₂ concentration, and a decrease in blood pH (see for example, Figure 2).

For another example, certain partial seizures are associated with a slight increase in arterial O₂ concentration, a decrease in arterial CO₂ concentration, and a slight increase in arterial pH (see for example, Figure 3).

In one embodiment, when the neurologic signal is a brain signal, the detection can be partially based on the observation that some seizure types are associated with sudden, transient increases in the amplitude at certain frequencies of cortical waves or with changes in their morphology (e.g., spike-slow wave complexes) (see for example, Figure 1).

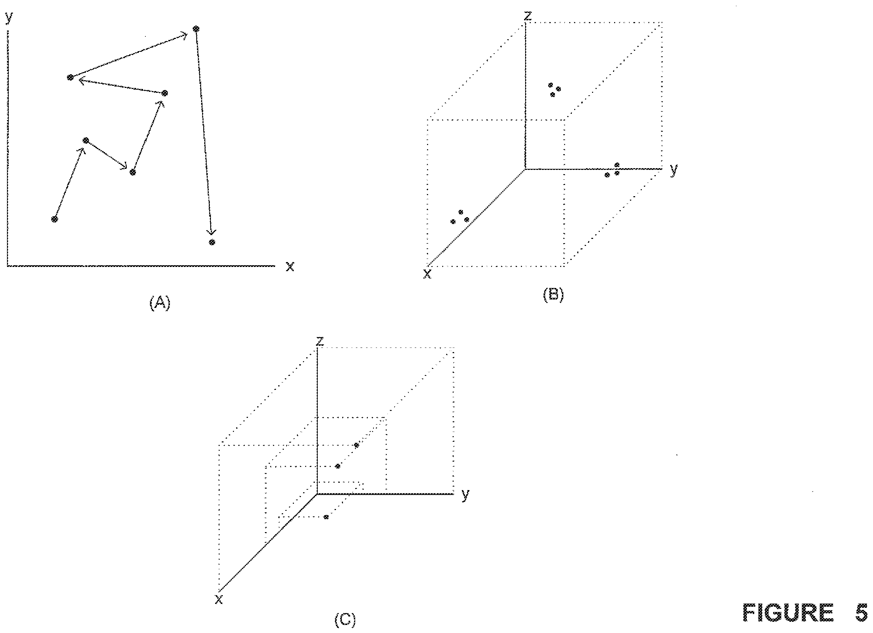

In one embodiment, when the neurologic signal is a kinetic signal, the detection can be partially based on the observation that some seizure types are associated with increases or decreased in the amplitude and velocity of movements the appearance of particular patterns or sequences of body or appendicular movements, cessation of movements or loss of postural tone or marked increased in body muscle tone as provided by electromyography (EMG), accelerometer, inclinometer, and/or actigraph outputs (see for example, Figure 1). EMG of anti-gravitatory muscles provides similar information to accelerometers or inclinometers about falls and in certain cases, EMG may replace them. For example, if a patient is in the recumbent position and has a generalized tonic-clonic seizure, the inclinometer and the accelerometer will not detect a fall but the EMG will (indirectly) by showing absence of muscle activity in antigravitatory muscles. This also applies to patients that at the onset of the generalized tonic-clonic seizure are either propped/supported. Falls during certain generalized tonic clonic seizures are caused by increases not decreases in postural muscle tone. Also, while muscle tome may be decreased or increased during partial seizures, the extent (number and type of muscle groups involved compared to generalized tonic-clonic seizures) allows for differentiation. Particular examples of kinetic signals relating to epileptic movements are shown in Figures 5-6.

U.S. Patent Application Publication 2009/0124870 to Arends et al. discloses a patient monitoring system using at least one heart rate sensor and a least one muscular tension sensor. The publication does not disclose acquisition or analysis of kinetic activity to monitor a patient. One or more of the embodiments of the present disclosure provide for detecting seizures through cardiac data (e.g., EKG) used in conjunction with motion data (e.g., accelerometer).

Figure 5A shows a two-dimensional (x,y) discrete trajectory of epileptic movements (low sampling rate is used to minimize computations but a continuous trajectory may be plotted). This plot contains spatial (in reference to a fiducial marker such as the patient's sternum) and temporal information (when a movement occurs and their order of occurrence) about body movements during an epileptic seizure. The arrows show the sequence of movements. Colors or shapes, instead of arrows may be used to track the temporal evolution of movements. When stereotypical the movement trajectory may be used as a template for detection using for example matched filtering. This plot may be also generated in 3-D.

Figure 5B shows a three-dimensional (x,y,z) discrete plot of epileptic movements The movements form clusters (3 in this example; the left most and lower most clusters are intended to illustrate interictal movements and the right most cluster, epileptic movements) that may have different shapes or dimensions for each patient. These clusters may be used (e.g., cluster analysis, principal component analysis) for detection, quantification, classification and/or validation of detection of seizures, and optionally as well as for logging, tracking the temporal evolution of seizures, and/or optimization of detection, quantification, classification, and/or of therapy. This plot contains only spatial information; temporal information may be added through the use of arrow or color or shape codes. When stereotypical the movement trajectory may be used as a template for detection using for example matched filtering.

Figure 5C shows a three-dimensional (x,y,z) discrete plot of epileptic movements; notice that one movement occurs only in 2-D (low sampling rate is used to minimize computations but a continuous trajectory may be recorded. This plot contains only spatial information (in reference to a fiducial marker such as the patient's sternum); temporal information may be added through the use of arrow or color or shape codes. When stereotypical the movement trajectory may be used as a template for detection using for example matched filtering.

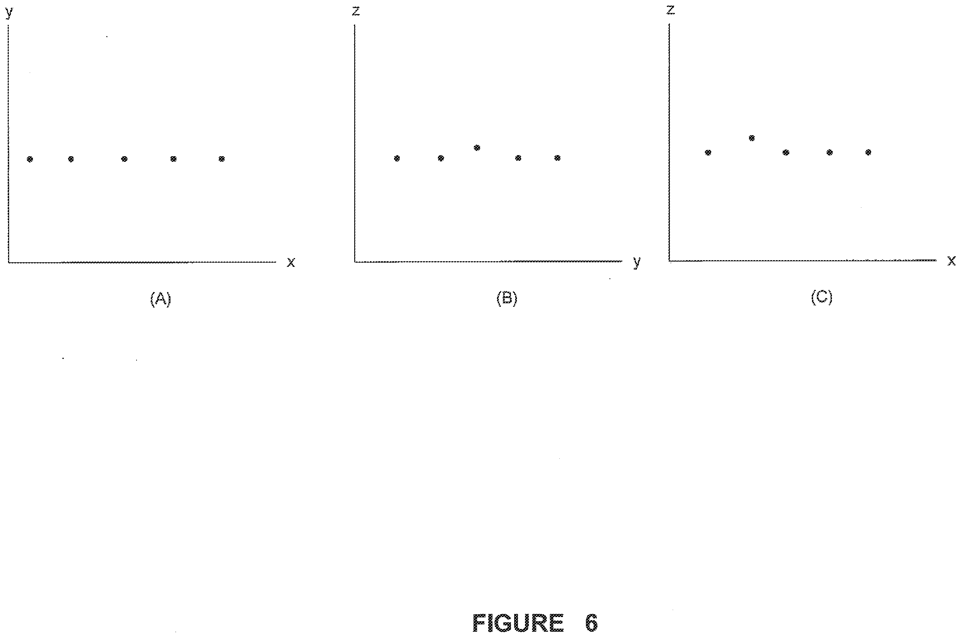

Figure 6 shows three two-dimensional, temporally cumulative plots of discrete movements during the clonic phase of a generalized (primarily or secondarily) tonic-clonic seizure. The first movement in the sequence is located closest to the x,y axes intersection and subsequent ones are plotted to the right of the preceding movement and in the order in which they occur. For ease of visualization there are 3 plots ((A) x,y; (B) y,z; (C) x,z). The vertical and horizontal axes provide information about amplitude and the horizontal axis also provides temporal information (e.g. inter-movement interval). In this illustration, the movements occur at equal time intervals and are periodic as is common in the clonic phase of a generalized seizure. When stereotypical the movement trajectory may be used as a template for detection using for example matched filtering.

U.S. Patent Application Publication 2009/0137921 to Kramer et al (Kramer) describes using accelerometer data to compare against previously stored motion data that are not confined to epileptic events. One or more of the embodiments of the present disclosure provide for detecting seizures through cardiac data (e.g., EKG) used in conjunction with motion data (e.g., accelerometer). Embodiments of the present disclosure may provide for detecting seizures using less specific motion data since the cardiac and motion data may be used to confirm each other.

Herein, one or more of the direction, speed/acceleration, trajectory (ID to 3D), pattern, and quality of movement may be termed a characteristic of movement. Such characteristics of movement may be determined for particular movements and used to distinguish among ictal, post-ictal, and interictal motor activity.

For example, absence seizures are typically correlated with a cessation of body movements and temporary but complete loss of responsiveness and awareness (see for example, Figure 4).

For another example, tonic-clonic seizures are associated with losses of responsiveness and awareness, and falls to the ground if the patient is standing at onset. Common characteristics of movement include a "spike" in the inclinometer's output at seizure onset (e.g., if the patient was standing, the seizure will cause him to fall), a quiet period of accelerometer output after seizure onset (e.g., the tonic phase), and a series of quasiperiodic "spikes" (e.g., at around 3 Hz) in accelerometer output after the tonic phase (e.g., the clonic phase), followed by cessation of body movements . The tonic phase presents with a marked increase in EMG activity in axial and appendicular muscles. Also, a "spike" in inclinometer output during or after the post-ictal phase may be seen (e.g., the patient rises after a fall at seizure onset) (see for example, Figure 2).

For yet another example, certain partial seizures are often correlated with a quiet period of accelerometer output after seizure onset (see for example, Figure 3), while others characterized by an increase in involuntary movements and vocalizations (e.g., so called "hypermotoric" seizures) .

Generally, movement characteristics, qualities, and loci are similar, if not stereotypical, among tonic-clonic seizures and certain partial seizures for a particular patient, and are also similar among patients with these seizure types. Thus, patterns can often be obtained and used for detection, quantification, and classification. However, in certain partial seizures, movements may differ not only between patients but also between seizures of the same patient.

In some embodiments of this disclosure, the number, type, and placement of motion sensors to be used in detecting, quantifying, and/or classifying movement can be based on (a) degree of movement similarity between seizures, (b) the signal-to-noise ratio of data from the locus or loci (e.g., body parts such as eyes, head, limbs, trunk, etc.), and/or (c) patient safety and device longevity considerations, among others. These considerations can be taken into account to maximize speed and/or accuracy of detection, quantification, and/or classifying, and/or performing this task or tasks in a monetary and/or computationally cost-effective manner.

For example, if a patient's tonic-clonic seizures are consistently preceded by a deviation of the head to the right, a single motion sensor (e.g., placed in this case on the head or over/in a neck muscle involved in the movement) may be sufficient to detect the motion and characterize the seizure. If the patient's seizures are characterized by sudden falls, again, a single device, placed in a body part that will have most acceleration or range of displacement, may be sufficient for seizure detection, quantification and classification purposes. If the patient's seizures are frequently secondarily generalized seizures, a plurality of devices, with at least one situated on each of the left and right sides of the body and/or with at least one situated on the upper and lower portions of the body may be desirable to provide sufficient sensitivity and specificity for seizure detection and characterization.

The choice of number of sensors, their type (e.g., whether they are sensitive to mechanical or electrical signal changes), and their placement can be optimized for each seizure type and patient.

In one embodiment, when the neurologic signal is a brain signal, the detection can be partially based on the observation that some seizure types are typically correlated with a decrease in responsiveness (see for example, Figure 1).

For yet another example, partial seizures can often be distinguished between simple and complex based on changes in the patient's responsiveness. Simple partial seizures are associated with preservation of awareness and memory for the events that occurred during the seizure and responsiveness may or may not be preserved, whereas complex partial seizures are invariably characterized by impairment in the patient's unawareness of their surroundings and anterograde amnesia spanning a certain time period (see for example, Figure 3). Responsiveness is tested by having the patient perform certain motor actions (e.g., press a button; raise an arm) and/or cognitive tasks (e.g., answer questions). Awareness may be tested by measuring a patient's ability to recollect events that occurred during a certain period of time or by administering memory tests. The number of words, images or events correctly recalled allow quantification of the degree of awareness (compared to an inter-ictal reference value). In one embodiment, the method further comprises providing a responsiveness test and awareness tests to assess patient's responsiveness and awareness, and characterizing the epileptic event based upon the speed and appropriateness or correctness of the responses to neuropsychologic tests.

For example, the following table shows conclusions that can generally be drawn from determinations of whether a patient remains responsive ("Responsive?") and/or remains aware ("Aware?") during a seizure.

| Responsive? | Aware? | Reasonable conclusion |
|---|---|---|
| Yes | Yes | Not a complex partial or secondarily generalized seizure |
| Yes or No | No | Complex partial seizure |
| No | Yes | Simple partial seizure interfering with ability to respond |
| No | No | Complex partial or primarily or secondarily generalized seizure |

The autonomic signal and the neurologic signal can be used to detect a seizure; to quantify its severity; to classify a seizure as to its type (e.g., absence, tonic-clonic, simple partial, complex partial); and/or to validate an identification or detection of a change of state as corresponding to a seizure.

The features of the two or more signals on which a detection or other action of the present disclosure is based may occur simultaneously or in any temporal relationship. In one embodiment, the temporal relationship between two signals is as set forth in Figures 1-4 and as described above. Relative temporal relationships between the body signals may be used identify, validate, classify, and/or quantify an epileptic event. Information relating to the timing of any two body signals, *e.g*., an increase in heart rate before, after, or substantially simultaneously with accelerometer data suggestive of a seizure, may be used to identify an epileptic event, validate an identification of an epileptic event, quantify an epileptic event's severity, intensity, or duration, and/or classify a seizure.

The various embodiments recited above may be also used to distinguish epileptic generalized from non-epileptic generalized seizures whose kinetic activity, but not patho-physiology, resembles that of epileptic seizures. Non-epileptic generalized seizures, also known as pseudo-seizures, psychogenic seizures, or hysterical seizures, are often misdiagnosed as epileptic at large cost to the patient, caregivers, and the health care system. A multimodal signal approach relying heavily on kinetic, autonomic and metabolic signals is ideally suited for diagnosing identifying and classifying seizures as non-epileptic given its high sensitivity and specificity and cost-effective (no hospital admission would be required as this disclosure's methods are implementable in small portable devices).

The following are a few examples of differences with high discriminatory values, one or more of which can be used to distinguish between epileptic generalized seizures and non-epileptic generalized seizures: a) The intensity of non-epileptic movements, unlike that of epileptic movements, waxes and wanes (crescendo-decrescendo pattern) throughout the event; b) Non-epileptic movements, unlike epileptic movements, are multi-directional or multi-planar, said changes in direction occurring very rapidly and in a random sequence. For example, vertical movements may give way to horizontal ones and these in turn to oblique or rotary or flapping movements; c) Joint movements in non-epileptic seizures, unlike in epileptic seizures, are incoherent or disorganized: while the right upper extremity is moving in the vertical plane at a certain speed and with certain amplitude and phase, the direction, speed, phase and amplitude of movement of the left upper extremity may be different at the same time; d) in non-epileptic seizures, unlike in epileptic seizures, co-activation of agonists and antagonists muscle groups is rarely seen: Co-activation of the abdominal and paraspinal muscles during an epileptic generalized tonic-clonic seizure keeps the torso straight while the sole activation of paraspinal muscles, a common observable in non-epileptic generalized seizures, manifests as an arched back; e) Involvement (in the form of movements) of certain body parts is commonly found in non-epileptic seizures while they are rarely if ever seen in epileptic generalized seizures; pelvic thrust, pelvic gyrations, and other pelvic movements are nearly pathognomic of non-epileptic seizures; f) Metabolic (lactic) acidosis occurs with epileptic generalized tonic-clonic seizures and not with non-epileptic generalized seizures.

Detection can be conducted by any appropriate technique. For example, each signal may be recorded, conditioned, and processed using hardware (e.g., DC or AC amplifiers), gains or amplification, filters and sampling rates appropriate for the spectral properties, and/or time-scale and characteristics of each signal. Each signal may be analyzed whole or after decomposition using suitable digital or analog signal processing techniques. The decomposition may be performed using any of the following techniques: Fourier transform based methods, wavelets, customized FIR or IIR filters, intrinsic time scale decomposition, wavelet transform maximum modulus, or any other technique which may decompose the signal based on its spectral properties, morphology or waveform, site of origin or generation, its position regarding a baseline, zero-crossings and circadian or ultradian rhythms. In the case of a decomposed signal none, one, or more of the components may be discarded if it is deemed of little value for detection of change of brain/body state. These data, as they stream through the system, may be analyzed in windows of appropriate length for each signal (e.g., signal-based customized window approach). This window corresponds to a foreground which may be referenced for quantitative purposes to a background, consisting of past data. The length of the background window may be determined by the properties of the signal under study and the time scale of the patterns or events which are the subject of detection. Any of these features or parameters may be adapted as needed to account for circadian or other influences to the signals

Although the above paragraph emphasizes hardware for signal conditioning and other tasks, the person of ordinary skill in the art is aware that software, firmware, or other implementations of one or more of the techniques discussed above may be used.

In one embodiment, the present disclosure relates to a method for detecting an epileptic event based upon a patient's cardiac signal and kinetic activity, comprising providing a cardiac signal indicative of the patient's heart beats; providing a kinetic signal indicative of a body movement of the patient; and detecting an epileptic event based upon the cardiac signal and the kinetic signal.

The cardiac signal may be electrical, acoustic, thermal, or any other cardiac signal detectable using certain equipment or tools. In one embodiment, the cardiac signal is provided by an electrocardiogram (EKG).

The kinetic signal can be provided by a device capable of recording any of the attributes inherent to movement such as amplitude, velocity, direction, trajectory and quality. In one embodiment, the kinetic signal is provided by an accelerometer, an inclinometer, or an actigraphic device. An actigraphic device or actigraph can be considered as being both an accelerometer and an inclinometer. An exemplary plot of trajectories is shown in Figure 5A. An exemplary plot of clusters of positions is shown in Figure 5B.

In one embodiment, the present disclosure relates to a method for detecting an epileptic event based upon a patient's cardiac signal and kinetic activity, comprising providing a kinetic signal indicative of a body movement of the patient; calculating based on the kinetic signal a kinetic score indicative of a correlation of said kinetic signal with an epileptic event; detecting an epileptic event based upon the patient's heart beat sequence; and providing an output indicative of an epileptic event based on the kinetic score..

In another embodiment, the method comprises providing a cardiac signal indicative of a cardiac activity of the patient; calculating based on the cardiac signal a cardiac score indicative of a correlation of said cardiac signal with an epileptic event; detecting an epileptic event based upon the patient's kinetic activity; and providing an output indicative of an epileptic event based on the cardiac score.

These are both examples of detecting an epileptic event based upon multimodal data, using a plurality of modes of data (e.g., a cardiac mode signal and a kinetic mode signal).

The kinetic signal indicative of a body movement of the patient can be as described above. A kinetic score can be calculated and/or the kinetic signal can be classified as either an epileptic event kinetic signal or a non-epileptic event kinetic signal based on the practitioner's knowledge (e.g., the practitioner is aware certain kinetic signals, e.g., inclinometer spikes, periods of increased accelerometer activity, periods of decreased accelerometer activity, periods of actigraph activity outside of normal ranges, timewise correlations of such signals, etc.), by prior correlation of a patient's kinetic signals with his or her seizures identified by autonomic (e.g., EKG), neurologic (e.g., EEG or direct or indirect clinical observation) endocrine, metabolic (e.g., pH), stress marker (e.g., cortisol) etc., or a combination thereof.

Imaging (e.g., video, thermography, etc.) and/or audio recordings of the patient may be used qualitatively or quantitatively to detect and/or validate the detection of seizures. Detection or validation may be made on- or off-line via human visual analysis or using algorithms that compare one or more of position, velocity, direction, or trajectory of movement of any body part during seizures to non-seizure movements. The time between consecutive movements, the total duration of epileptic movements, and/or their quality (e.g., jerky or smooth) may be also used for detection and/or validation of seizures.

Detecting a possibility of an epileptic event based upon the patient's heart beat sequence can make use of the cardiac-based seizure detection approaches discussed above. For example, noting an increase in the patient's heart rate relative to an interictal reference value is one embodiment of "detecting an epileptic event," *e.g*., a period of increased likelihood of a seizure.

Upon classifying the kinetic signal and detecting the possibility of the epileptic event based upon the patient's heart beat sequence, an output indicative of an epileptic event can be provided if the kinetic signal is classified as an epileptic event kinetic signal; and an output indicative of the non-occurrence of an epileptic event can be provided if the kinetic signal is classified as a nonepileptic event kinetic signal. This method can validate a cardiac-based seizure detection by use of kinetic signals.

In another embodiment, the present disclosure relates to a method for detecting an epileptic event based upon a patient's cardiac signal and kinetic activity, comprising providing a kinetic signal indicative of a body movement of the patient; classifying the kinetic signal as either an epileptic event kinetic signal or a nonepileptic event kinetic signal; detecting an epileptic event based upon changes in the patient's heart beat sequence; confirming the detecting if said kinetic signal is classified as an epileptic event kinetic signal; overriding the detecting if said kinetic signal is classified as a nonepileptic event kinetic signal; and providing an output indicative of an epileptic event only if the detecting is confirmed.

In yet another embodiment, the present disclosure relates to a method for detecting a tonic-clonic epileptic seizure whether primarily or secondarily generalized (*i.e*., whether the seizure emerges in both hemispheres of the brain at substantially the same time (primary) or whether it emerges at a particular focus and then spreads (secondary) based upon two or more of a patient's body signals, comprising: providing at least two body signals selected from the group consisting of a cardiac signal indicative of the patient's heart beats; an accelerometer signal indicative of the patient's movement; an inclinometer signal indicative of the patient's body position; an actigraph signal indicative of the patient's movement, body position, or both; a respiratory signal indicative of the patient's respiration; a skin resistivity signal indicative of the patient's skin resistivity; an blood gas signal indicative of the patient's blood oxygen content, carbon dioxide content, or both; a blood pH signal indicative of the patient's blood pH; an isometric force signal indicative of the patient's muscle activity; a sound signal indicative of the patient's oral utterances or vocalizations; an ocular signal indicative of the patient's eye position and movements; a responsiveness signal indicative of the patient's responsiveness; and a stress marker signal indicative of at least one stress marker of the patient; and detecting the generalized tonic-clonic epileptic seizure based upon the timewise correlation of two features, one feature being of each of the at least two body signals. The term, and concept of, "responsiveness" as used in reference to the embodiments described herein, has a motor and a cognitive component which may be strongly correlated or dissociated; further the motor component may be in the form of a simple response (*e.g*., withdrawal of a limb from a pain source) or complex (*e.g*. drawing a triangle in response to a command). Consequently, responsiveness may be tested using simple stimuli (*e.g*., acoustic in the form of a loud noise or sensory in the form of a pinprick) or complex (*e.g*., complex reaction time tests; questions probing knowledge, judgment, abstraction, memory, etc.). In this disclosure, when "responsiveness" is tested using complex stimuli, "awareness" is being probed and therefore in that case these terms/concepts are used interchangeably. The meaning of "responsiveness" is thus, context dependent: if the objective is to determine if a patient generates simple motor responses or movements, the term "responsiveness" may be used and if it is to test the presence and quality of complex responses, "awareness" may replace responsiveness.

As used herein, "spectral analysis" encompasses spectral analyses using at least one of the known methods (e.g., Fourier-based, wavelet based; multifractal spectral, etc.) of cardiac activity or body movements. Spectral analysis techniques are known to the person of ordinary skill in the art and can be implemented by such a person having the benefit of the present disclosure. Spectral analysis may be discrete or continuous. Spectral analysis of a cardiac activity can comprise spectral analysis of heart rate or individual beats' EKG complexes, among others.

Patient indices or features can be a value derived directly from the signal relating to the first cardiac activity or the signal relating to the first body movement. For example, one or more cardiac indices or features can be derived from a cardiac activity signal over one or more periods of time. For example, a foreground heart rate over a relatively short time period (e.g., 5-30 sec) and a background heart rate over a longer time period (*e.g*.., 30-600 sec) can both be derived from a cardiac activity signal. For another example, an accelerometer or inclinometer mounted on a patient's body can give information about the patient's (and/or parts of his body) movements and body position.

The patient features can also be used in a determination of an epileptic event. For example, the cardiac activity and/or body movement can be analyzed by determining one or more cardiac features and/or kinetic features to determine an occurrence of an epileptic event, a non-occurrence of an epileptic event, or a probable occurrence of an epileptic event.

In one embodiment, triggering additional test(s) can be based on at least one of a patient's cardiac activity and the patient's body movement upon a finding that the cardiac activity and/or body movement are indicative of a possible epileptic event. For example, if cardiac activity and/or body movement features clearly indicate an epileptic event with high confidence, triggering additional test(s) need not be performed; but if the cardiac activity and/or body movement features are outside their interictal reference value ranges but have values that give only low confidence of an epileptic event, triggering additional tests can be performed to provide additional information about the patient's condition to indicate whether he or she is suffering an epileptic event or not.

For another example, the patient's cardiac activity at a first time may indicate an epileptic event, and the patient's body movement at a second time and in a particular region of the body may indicate an epileptic event, but if the two times differ, or the body movement is in a different region of the body, or changes in their characteristics (*e.g*., rate, morphology, pattern, etc.) are discordant with declaring the epileptic event, consideration of cardiac activity and body movement may lead to low confidence of an indication of an epileptic event, and in response thereto, triggering of additional test(s) and/or consideration of additional body signals may be desirable. In other words, there may be a low absolute value of correlation (e.g., a correlation between about -0.4 and 0.4) between the patient's cardiac activity and the patient's body movement that would prevent highly confident determination of an epileptic event. The triggered additional test(s) may provide enough additional information to make a highly confident determination of an epileptic event (or the non-occurrence of an epileptic event).

Generally, two parameters can be considered highly correlated if the coefficient of correlation is greater than about 0.7, and lowly correlated if the coefficient of correlation is less than about 0.4. Two parameters can be considered highly anticorrelated if the coefficient of correlation is less than about -0.7, and lowly anticorrelated if the coefficient of correlation is greater than about -0.4. One example of parameters/situations that can be considered to be anticorrelated includes an appearance of tachycardia with a disappearance of body movement. Other examples that can be considered to be anticorrelated are a strong body movement with either a substantially unchanged heart rate or a decreased heart rate. The example with the substantially unchanged heart rate can be considered a low anticorrelation, and the example with the decreased heart rate can be considered a high anticorrelation.

Another pair of examples to consider is the correlation between body movement and first derivative of heart rate in an epileptic event vs. in exercise. Generally, the first derivative of heart rate is greater in an epileptic event than in exercise, *i.e.,* body movement and the first derivative of heart rate can be considered more highly correlated in epileptic events than in exercise.

The presence of either high or low correlation (or anti-correlations) may be used in this disclosure to determine the occurrence of an epileptic event and trigger an action(s)or to determine that an epileptic event is not occurring or did not occur. The first and second cardiac activity may be the same (in other words, triggering can be of a second iteration of a test that reported the first cardiac activity as a result of a first iteration, giving a more current value of the cardiac activity), or they may be different. In one embodiment, the first cardiac activity is heart rate or heart rate variability, and the second cardiac activity is heart beat morphology.

Similarly, the first and second body movement may be the same, or they may be different.

A "test" is used herein to refer to any assay of the patient's cardiac activity, body movement, responsiveness, awareness, or a spectral analysis thereof. The product of a test can be considered a signal, and a signal can be considered as resulting from a test. A test of the second cardiac activity may use substantially the same data source, data processing, and/or related techniques as are used in receiving the signal relating to the first cardiac activity. In another embodiment, the techniques may differ. For example, the first cardiac activity can be heart beat morphology determined by electrocardiography (EKG), and the second cardiac activity can be heart beat morphology determined by phonocardiography (PKG).

Similarly, a test of the second body movement may, but need not, use substantially the same data source, data processing, and/or related techniques as are used in receiving the signal relating to the first body movement.

The concept of first and second cardiac activity or first and second body movement is also applicable to responsiveness and awareness. For example responsiveness activity may be a reflex movement such as withdrawal from a source of painful stimuli and a second responsiveness activity may be a complex movement such as that required to draw a triangle. Different tests of varying levels of complexity may be administered to test responsiveness as defined in this disclosure.

The particular triggered test(s) may be selected based at least in part on the first cardiac activity, the first body movement, or both.

In one embodiment, determining is based on at least one of a finding the patient's awareness differs from a reference responsiveness level, a finding the patient's awareness differs from a reference awareness level, a finding the patient's second cardiac activity includes a characteristic suggestive of an epileptic event, a finding the spectral analysis of the patient's second cardiac activity includes a characteristic suggestive of an epileptic event, and a finding the spectral analysis of the patient's second body movement includes a characteristic suggestive of an epileptic event.

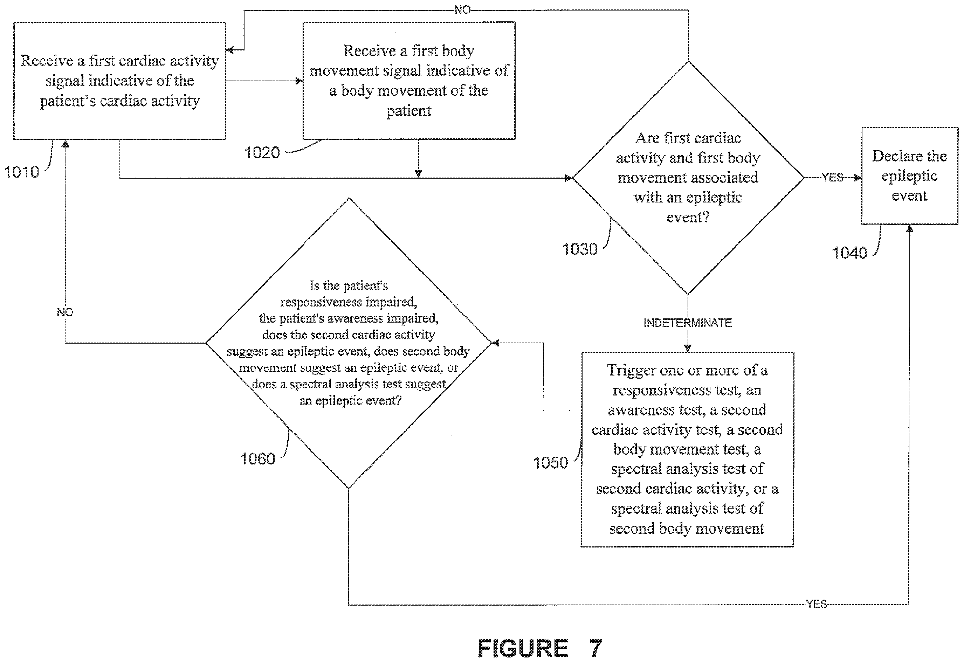

Figure 7 shows a flowchart depicting one embodiment of a method according to the present disclosure. A cardiac activity signal indicative of the patient's cardiac activity is received at block 1010 and/or a body movement signal indicative of a body movement of the patient is received at block 1020.

Thereafter, a determination is made in block 1030 whether cardiac activity and body movement are associated with an epileptic event. If no, flow returns to the receiving blocks 1010-1020. If yes, an epileptic event is declared at block 1040. However, if no determination can be made, flow moves to block 1050, where one or more of a responsiveness test, an awareness test, a second cardiac activity test, a second body movement test, a spectral analysis test of the second cardiac activity, or a spectral analysis test of the second body movement, are triggered.

Thereafter, a determination is made in block 1060 whether the patient's responsiveness, awareness, second cardiac activity, second body movement, and/or spectral analysis of second cardiac activity or second body movement are indicative of an epileptic event. If no, flow returns to the receiving blocks 1010-1020. If yes, an epileptic event is declared at block 1040.

Alternatively or in addition to declaring an epileptic event, further actions can be performed. In one embodiment, the method further comprises classifying the epileptic event based upon at least one of the first cardiac activity, the first body movement, the responsiveness, the awareness, the second cardiac activity, the second body movement,, the spectral properties of the second cardiac activity, the spectral properties of the second body movement, and two or more thereof.

Classifications of epileptic events can be generally based on the information shown in Figures 1-4 and the discussion herein. Classifications can also be based in part on observations of stereotypical seizures of a particular patient. Not all seizures that a clinician would recognize as being of a particular type may exhibit all the properties discussed herein, and thus, not all may be amenable to classification by the methods described herein, but a substantial majority are expected to be amenable to classification by the methods described herein.

In one embodiment, the epileptic event is classified as a generalized tonic-clonic seizure when the following occur in a patient in a first, non-recumbent position: the first body movement comprises a fall from the first, non-recumbent position, wherein (i) the fall is associated with a loss of responsiveness, a loss of awareness, or both; and (ii) the fall is followed by generalized body movements.

Falls to the ground associated with a primarily or secondarily generalized tonic-clonic, generalized tonic, generalized clonic-tonic-clonic seizure or generalized atonic seizure are distinguishable from those caused by tripping or slipping by the absence of protective/defensive actions (e.g., breaking the fall with the arms) and other features such which body part(s) is(are) first on contact with the ground.

Primarily generalized seizures usually result in synchronous bilateral movements of equal amplitude, with maintenance of head and eyes on the midline. Secondarily generalized seizures usually manifest at onset with unilateral movements of limbs, head, eyes, or trunk.

In one embodiment, the generalized body movement comprises a rhythmic body movement. Alternatively or additionally, the generalized body movements can comprise flexion and extension of joints and/or can have a frequency of about 3 Hz at some time during the epileptic event. In another embodiment, the rhythmic movement is temporally associated with an epileptiform discharge.

Body movement can allow classification of an epileptic event as to primarily generalized or secondarily generalized. Specifically, the epileptic event can be classified as primarily generalized if body movements are synchronous and of equal amplitude on both sides of the body, and as secondarily generalized if not.

In a further embodiment, the epileptic event is classified as a generalized tonic-clonic seizure when recovery of awareness follows recovery of responsiveness, provided at least one of the key identifiers (e.g., loss of postural tone or diffuse increase in muscle tone or rhythmical body movements) have occurred.

In one embodiment, the epileptic event is classified as an atonic seizure when the following occur in a patient in a first, non-recumbent position:
i) a body movement comprises a fall from the first, non-recumbent position, wherein the fall is associated with a loss of responsiveness, a loss of awareness, or both; and
(ii) the patient shows a significant reduction in body movements below a reference value after the fall, a significant reduction in muscle tone below a reference value after the fall, or both.

Typically, the significant reductions in body movements and/or muscle tone commonly seen in atonic seizures are not caused by changes in heart or respiratory activity.

In one embodiment, the epileptic event is classified as tonic when the following occur to a patient in a first, non-recumbent position: an increase in muscle tone above a reference value, a loss of responsiveness, and an absence of generalized movements.

In a further embodiment, the epileptic event is classified as tonic when recovery of awareness follows recovery of responsiveness, provided it has been associated with loss of responsiveness or awareness.

In one embodiment, the epileptic event is classified as a complex partial seizure based upon a finding the patient's cardiac activity is associated with impaired awareness and is not associated with a fall or at some point in time with generalized rhythmical body movements; and the epileptic event is classified as a simple partial seizure based upon a finding the patient's cardiac activity is not associated with impaired awareness and is not associated with generalized rhythmical body movements.

In one embodiment, the event is classified as syncope, when at least one of the following occur: the body movement comprises a fall from a non-recumbent position and the fall is associated with a loss of responsiveness or a loss of awareness, and recovery of responsiveness or recovery of awareness occurs immediately after the fall, or when the body movement comprises a fall from a recumbent position, there is marked decrease in heart rate or a brief transient cessation of heart beats (asystole).

Epileptic events can be determined or classified in view of the patient's body position. For example, an epileptic event when the patient is in a decubitus position (lying down) may be determined from an observation of transient loss of muscle tone in antigravitatory muscles (e.g., paraspinal; quadriceps), followed by transient increase in muscle tone in agonist and antagonist muscle groups (e.g., paraspinal and abdominal recti; quadriceps and hamstrings), which in turn is followed by generalized rhythmical muscle contractions (typically with a frequency of 3 Hz and/or 10-12 Hz at some time during the event).

For another example, an epileptic event when the patient is in a seated position may be determined using both electromyography (EMG) signals and accelerometer signals.

The one or more of the first cardiac activity, the second cardiac activity, the first body movement, the second body movement, the responsiveness, and the awareness can be provided by any known technique. In one embodiment, at least one of the first cardiac activity and the second cardiac activity is sensed by at least one of an electrocardiogram (EKG), phonocardiogram (PKG), apexcardiography, blood pressure monitor, and echocardiography. The body movement can be sensed by any known technique. In one embodiment, at least one of the first body movement and the second body movement is sensed by an accelerometer, an inclinometer, an actigraph, an imaging system, a dynamometer, a gyroscope, electromyography (EMG), or two or more thereof.

In certain circumstances, the method can make a false positive determination of an epileptic event, *i.e.,* determine an epileptic event based on the signals and tests described above when no epileptic event (as may be determined using direct/invasive recording of electrical activity at/near the epileptogenic zone, observation by a skilled practitioner, or other techniques known to the person of ordinary skill in the art) occurred. In one embodiment, the method further comprises receiving an indication that the determined epileptic event was not an actual epileptic event. Such indications may include, but are not limited to, the first body movement is a fall but the fall is not characteristic of an epileptic fall; the generalized body movements are not rhythmical and bilaterally synchronous; the generalized body movement have a frequency substantially different from 3 Hz or a variable frequency; the generalized body movements change direction, pairs of agonist-antagonist muscles, and/or movements in different directions occur simultaneously in two or more joints; the change in cardiac activity, cardiac activity morphology, cardiac spectral analysis, apexcardiography, or echocardiography is not characteristic of epileptic seizures.

Similarly, in one embodiment, the method further comprises receiving an indication of a false negative, *i.e.,* an indication an epileptic event occurred but no determination thereof was made.

The indication may be based at least in part on input from the patient, a caregiver, or a medical professional, and/or on quantification or characterization of one or more body signals. The indication may be provided at the time of the false determination or later.

A false determination (whether positive or negative) may render it appropriate to modify the body signals or analyses used in making future determinations. In one embodiment, the method further comprises reducing a likelihood of a future determination of a false positive epileptic event based at least in part on one or more of the first cardiac activity, the first body movement, the responsiveness, the awareness, the second cardiac activity, the second body movement, the spectral analysis of the second cardiac activity, or the spectral analysis of the second body movement, in response to the indication. In another embodiment, the method further comprises reducing a likelihood of a future determination of a false negative epileptic event based at least in part on one or more of the first cardiac activity, the first body movement, the responsiveness, the awareness, the second cardiac activity, the second body movement, the spectral analysis of the second cardiac activity, or the spectral analysis of the second body movement, in response to the indication.

When an epileptic event is determined, the method can further comprise one or more of logging the occurrence and/or time of occurrence of the seizure; providing a warning, alarm or alert to the patient, a caregiver or a health care provider; providing a therapy to prevent, abort, and/or reduce the severity of the seizure; assessing one or more patient parameters such as awareness or responsiveness during the seizure; assessing the severity of the seizure; identifying the end of the seizure; and assessing the patient's post-ictal impairment or recovery from the seizure. "Recovery" is used herein to encompass a time after seizure onset and/or seizure end when the patient's parameters are returning to baseline. Other examples include, but are not limited to, logging one or more of a time of onset of the epileptic event, a time of termination of the epileptic event, a severity of the epileptic event, an impact of the epileptic event, an interval between the epileptic event and the most recent preceding epileptic event, an epileptic event frequency over a time window, an epileptic event burden over a time window, time spent in epileptic events over a time window, or a type of epileptic event.

To reduce the rate of false positive detections or for other reasons, in one embodiment, the method further comprises
recording one or more of the patient's reference body movement or movements, reference cardiac activity, reference responsiveness level, reference awareness level, reference cardiac activity, reference spectral analysis of the cardiac activity, or reference spectral analysis of the body movement during one or more interictal activities at one or more times when the patient is not suffering an epileptic event, to yield recorded data not associated with an epileptic event; defining one or more interictal activity reference characteristics from the recorded data; and overruling the determination of the epileptic event based at least in part on finding the patient's first body movement, first cardiac activity, responsiveness level, awareness level, second cardiac activity, second body movement, spectral analysis of the second cardiac activity, and spectral analysis of the second body movement matches the one or more interictal event reference characteristics.

The interictal activities at one or more times when the patient is not suffering an epileptic event can include different activities (*e.g*., walking vs. running vs. swimming, etc.), and can alternatively or in addition include the same activity at different times of day, week, month, or year, or under different external circumstances (*e.g*., walking at sea level vs. walking at higher altitude, etc.).

The overruling of a determination of an epileptic event may be made with some probability between zero and one. The overruling may be made according to a permanent or semipermanent rule or on a case-by-case basis. The references may be stored in a library on a per-patient, per-seizure type, or per-population basis.

In one embodiment, the overruling may involve the triggering of one or more additional test(s). Such further triggering may allow more accurate determination of epileptic events.

Recording one or more of the patient's reference body movement or movements, reference cardiac activity, reference responsiveness level, reference awareness level, reference cardiac activity, reference spectral analysis of the cardiac activity, or reference spectral analysis of the body movement during epileptic event may allow overruling of false negative or false positive determinations.

The body movement during one or more interictal activities can include at least one of a movement of a part of the body(*e.g*., the eyes or eyelids), a movement of a limb (e.g., an arm), a movement of a part of a limb (*e.g*., a wrist), a direction of a movement, a velocity of a movement, a force of a movement, an acceleration of a movement, a quality of a movement, an aiming precision of a movement, or a purpose or lack thereof of a movement.

The likelihood of a patient suffering an epileptic event may change at different times and/or under different conditions. In one embodiment, a plurality of interictal event reference characteristics are defined which differ from one another based on one or more of the time of day of the recording, the time of week of the recording, the time of month of the recording, the time of year of the recording, the type of activity, changes in the patient's body weight or body mass index, changes in the patient's medication, changes in the patient's physical fitness or body integrity, state of physical or mental health, mood level or changes in the patient's mobility. Alternatively or in addition, a plurality of interictal event reference characteristics in a female patient can be defined in reference to the menstrual cycle and/or to pregnancy. Alternatively or in addition, changes in the patient's environment may change the likelihood of the patient suffering an epileptic event.

In a further embodiment, the overruling is based at least in part on one or more of the plurality of interictal event reference characteristics.

Any characteristic of the one or more interictal events may be considered. In one embodiment, the one or more characteristics are patterns or templates.

It may be desirable in certain embodiments to adapt at least one of a reference value on one or more of the body movement, the cardiac activity, the responsiveness level, the awareness level, the second cardiac activity, the second body movement, and the spectral analysis of cardiac activity or body movement, based upon one or more determinations that the specificity of past detections was above or below a specificity measure, the sensitivity of past detections was above or below a sensitivity measure, the speed of detection defined as the time elapsed between the occurrence of the first body signal change indicative of the onset of the seizure and the issuance of the detection, the cost of the therapy was below or above a cost measure, the patient's tolerance of the therapy was below an acceptable tolerance, the adverse effects were above an acceptable level, or the patient's disease state was below or above a first disease state threshold. Positive predictive value or negative predictive value may be used in addition to or instead of specificity or sensitivity.

As should be apparent, a single "threshold" can be mathematically defined in a number of ways that may be above or below a particular value of a particular parameter. For example, an elevated heart rate can be defined, with equal validity, as a heart rate above a threshold in units of beats/unit time or an interbeat interval below a threshold in units of time. More than one "threshold" may be used to optimize specificity, sensitivity or speed of detection.

For example, the method can further comprise determining one or more of a specificity of past detections, a sensitivity of past detections, a speed of past detections, a cost of a therapy for epileptic events, a patient's tolerance of a therapy for epileptic events, and a disease state of the patient; and loosening at least one constraint on one or more of the body movement, the cardiac activity, the responsiveness test, the awareness test, the second cardiac activity test, the second body movement test, and the spectral analysis of second cardiac activity or second body movement based upon one or more determinations that the specificity of past detections was above a first specificity threshold, the sensitivity of past detections was below a first sensitivity threshold, the speed of detection was below a first speed of detection threshold, the cost of the therapy was below a first cost threshold, the patient's tolerance of the therapy was below a first tolerance threshold (i.e., the patient can tolerate more detections or actions performed in response to detections), and the patient's disease state was below a first disease state threshold; or tightening the at least one constraint based upon one or more determinations that the specificity of past detections was below a second specificity threshold, the sensitivity of past detections was above a second sensitivity threshold, the speed of detection was above an acceptable threshold for efficacy of therapy and safety of the patient, the cost of the therapy was above a second cost threshold, the patient's tolerance of the therapy was above a second tolerance threshold (*i.e*., the patient cannot tolerate more detections or actions performed in response to detections), and the patient's disease state was above a second disease state threshold.

In another embodiment, the disclosure can be used for the detection of generalized tonic-clonic seizures. A "generalized tonic-clonic seizure" is used herein to refer to a primarily or secondarily generalized seizure that features at least one tonic, clonic, or both tonic and clonic phase. Myoclonic seizures are included in this definition. At onset or at some point during the generalized tonic-clonic seizure, at least a majority of the body muscles or joints are involved. "Body muscle" is used herein to refer to those capable of moving joints, as well as muscles of the eyes, face, orolaryngeal, pharyngeal, abdominal, and respiratory systems.

In one embodiment, the present disclosure relates to a method, comprising:
receiving at least two body signals selected from the group consisting of a signal relating to a first body movement, a signal relating to a first cardiac activity, a responsiveness signal, an awareness signal, a signal relating to a second cardiac activity, a signal relating to a second body movement, a spectral analysis signal relating to the second cardiac activity, and a spectral analysis signal relating to the second body movement;
determining an occurrence of a generalized tonic-clonic epileptic seizure, the determination being based upon the correlation of at least two features, at least one feature being of each of the at least two body signals, wherein:
   the feature of the first cardiac activity signal is an increase in the patient's heart rate above an interictal reference value;
   the feature of the first body movement signal is at least one of (i) an increase in axial or limb muscle tone substantially above an interictal or exercise value for the patient, (ii) a decrease in axial muscle tone in a non-recumbent patient, below the value associated with a first, non-recumbent position, (iii) fall followed by an increase in body muscle tone, or (iv) a fall followed by generalized body movements;
   the feature of the responsiveness signal is a decrease in the patient's responsiveness below an interictal reference value;
   the feature of the awareness signal is a decrease in the patient's awareness below an interictal reference value;
   the feature of the second cardiac activity signal is a correlation with an ictal cardiac activity reference signal;
   the feature of the second body movement signal is a correlation with an ictal body movement reference signal;
   the feature of the spectral analysis signal of the second cardiac activity is a correlation with an ictal cardiac activity spectral analysis reference signal; or
   the feature of the spectral analysis signal of the second body movement is a correlation with an ictal body movement spectral analysis reference signal;
      and
performing a further action in response to the determination of the occurrence of the epileptic event.

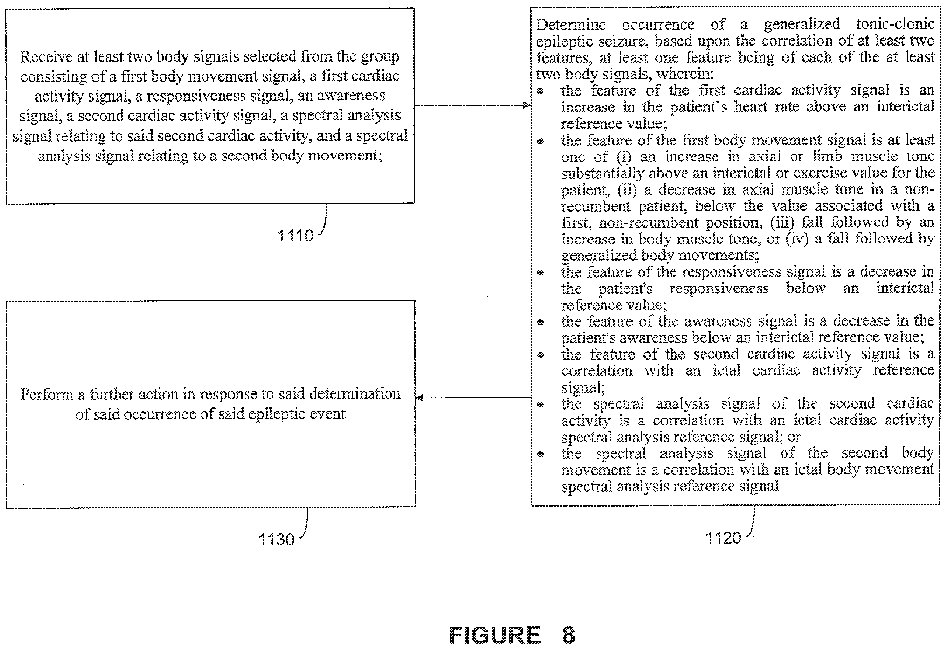

Figure 8 depicts one embodiment of this method. Figure 8 depicts a receiving step 1110, a determining step 1120, and a performing step 1130.

In one embodiment, the correlation has a high absolute value and is either positive or negative. E.g. the correlation may be positive, such as with a value greater than 0.7, 0.75, 0.8, 0.85, 0.9, or 0.95, or negative, such as with a value less than -0.7, -0.75, -0.8, -0.85, -0.9, or -0.95.

The further action may comprise one or more of logging the occurrence and/or time of occurrence of the seizure; providing a warning, alarm or alert to the patient, a caregiver or a health care provider; providing a therapy to prevent, abort, and/or reduce the severity of the seizure; assessing one or more patient parameters such as awareness or responsiveness during the seizure; assessing the severity of the seizure, identifying the end of the seizure; and assessing the patient's post-ictal impairment or recovery from the seizure.

The various signals can be provided by any appropriate technique and their features referred to above can likewise be measured. For example, in one embodiment, the correlation of the second cardiac activity signal with the ictal cardiac activity reference signal comprises a match to an ictal cardiac activity template;
the correlation of the second body movement signal with the ictal body movement reference signal comprises a match to an ictal body movement template;
the correlation of the spectral analysis signal of the second cardiac activity with the ictal cardiac activity spectral analysis reference signal comprises a match to an ictal cardiac activity spectral analysis pattern or template; or
the correlation of the spectral analysis signal of the second body movement with the ictal body movement spectral analysis reference signal comprises a match to an ictal body movement spectral analysis pattern or template. Aspects of the signals and their features may include, among others, a body movement signal further comprising an indication of a fall prior to the indication of the tonic or clonic activity.

In one embodiment, a tonic-clonic seizure can be further characterized as secondarily generalized if the first body movement signal does not comprise synchronous movement of all body muscles with equal amplitude or velocity prior to an indication of tonic or clonic activity.

In one embodiment, the end of the generalized tonic-clonic epileptic seizure can be indicated when at least one of the body signals trends toward an interictal reference value, range, or pattern of the body signal.

In one embodiment, the method further comprises indicating the beginning of a post-ictal period based upon the appearance of at least one post-ictal feature of at least one the body signal, wherein:
the post-ictal feature of the first cardiac signal or the second cardiac signal is a decrease in the patient's heart rate below an ictal reference value;
the post-ictal feature of the first body movement signal or the second body movement signal is a decrease in the patient's muscle tone or movement below an ictal reference value;
the post-ictal feature of the responsiveness signal is an increase in the patient's responsiveness above an ictal value and below an inter-ictal reference value; or
the post-ictal feature of the awareness signal is an increase in the patient's awareness above an ictal value and below an inter-ictal reference value.

The term "post-ictal," is not necessarily limited to the period of time immediately after the end of the primarily or secondarily generalized tonic-clonic epileptic seizure and is not limited to this type of seizure but also encompasses partial seizures (*e.g*., all complex and certain simple partial and absence seizures). Rather, it refers to the period of time during which at least one signal has one or more features that differs from the ictal and inter-ictal reference values that indicates one or more of the patient's body systems are not functioning normally (e.g., as a result of the seizure or of an injury suffered during the seizure) but are not exhibiting features indicative of a seizure.

In one embodiment, the end of the post-ictal period can be indicated when each of the post-ictal features is outside the range of values associated with the ictal and post-ictal states. In another embodiment, the end of the post-ictal period can be indicated when at least one of the post-ictal features is outside the range of values associated with the ictal and post-ictal states. In this embodiment, the onset and termination of the post-ictal period may be partial when all features have not returned to interictal reference values or complete when all features have. This distinction (partial vs. complete) has important therapeutic (the patient may require treatment until all body signals have fully recovered to inter-ictal values), safety (the patient's mortality and morbidity risks may remain increased until all body signal have fully recovered to inter-ictal values) and predictive implications (the probability of occurrence of the next seizure and time to it (inter-seizure interval) may depend on recovery of one more body signals to their interictal value.

It should also be borne in mind that different features are expected to return to their interictal reference values at different times. For example, from kinetic and brain electrical perspectives, a seizure can be defined as having ended when abnormal movements and abnormal EEG cease. These events typically take place before the patient's heart rate returns to baseline. Further, it may take a few minutes after abnormal movements and abnormal EEG end for cognition and responsiveness to return to baseline; up to about 30 min for awareness to return to baseline; and about 30-45 min for blood lactic acid concentration to return to baseline.

In a further embodiment, this method further comprises indicating the end of the postictal period and the beginning of the inter-ictal period when the values of at least one of the post-ictal features changes to being within the range of reference body signal values or behavior associated only with the inter-ictal period wherein:
the cardiac signal returns to a heart rate within a range indicative of an interictal state for said patient
the accelerometer signal of the patient's movement velocity, amplitude or number of movements per unit time returns to values indicative of an inter-ictal state for said patient;
the accelerometer signal is a movement pattern including inter-movement intervals or trajectory indicative of that patient's inter-ictal period
the respiratory signal (e.g., rate, tidal volume, minute volume, and pattern) returns to a range indicative of the inter-ictal state for that patient; the responsiveness signal returns to its range of inter-ictal values for that patient; and
the patient's awareness returns to inter-ictal ranges for the patient.

The changes in signal features (e.g., responsiveness, awareness, heart activity, respiratory activity, etc.) during the transitions (e.g., inter-ictal to ictal, ictal to post-ictal and post-ictal to interictal) that make up the epileptic cycle, may or may not occur simultaneously or synchronously; certain signal feature values change ahead or behind others. Thus, using these signal features, the transitions may be qualitatively classified into (a) partial or complete; (b) quantitatively as the fraction of signal features (numerator) that transitioned into or out of the state over the total number of signal features that have been observed (denominator).

For example, if only 2/4 signal feature values indicative of the transition from ictal to postictal or from post-ictal to inter-ictal have reached values within the range of the new state, the is classified as partial assigned an score of 0.5 and declared complete only when all (e.g., 4/4 in this example) signal features values are within the range of indicative of the new state at which time the transition is deemed completed.

The transitions may be also quantified using the: a) magnitude of the change in feature signal values measured for example as the increase in seizure energy (see Osorio et al, Epilepsia 1998, 2001) as compared to its inter-ictal value, or the percent of incorrect responses to a complex reaction time test compared to the responses in the inter-ictal state, or the lengthening in response time regardless of correctness of responses (see, *e.g*., US 12/756,065, filed April 7, 2010, which is hereby incorporated herein by reference) compared to that recorded in the inter-ictal state for that patient; b) rate of change in the signal features measured for example as the time to peak value change measured from the onset time of the transition or the time to first error in a complex reaction time compared to those obtained in the inter-ictal period; c) duration (e.g., in seconds) of the state change from the onset of the inter-state transition to the beginning of the transition from the present state (e.g., ictal) to another state (e.g. post-ictal). These metrics may be used to *e.g*., assess the disease state (e.g., the duration and magnitude of the ictal state are increasing over time) and also the efficacy of therapeutic interventions. Shortening the magnitude of the changes (e.g., degree of unresponsiveness) in signal feature values from the inter-ictal range to the ictal value or the transition time between the post-ictal and interictal periods provide evidence that the therapy is beneficial while increases in the magnitude or duration of the changes in feature signals from the inter-ictal range to ictal value or a lengthening of the transition from the post-ictal to the interictal state are evidence of an adverse therapeutic effect. The qualitative and quantitative categorization of the various states and of their transitions is applicable to all seizures and epilepsy types and also to other states and inter-state transitions. In another embodiment, the present disclosure relates to the detection of partial seizures. In one embodiment, the present disclosure relates to a method, comprising:
receiving at least two body signals selected from the group consisting of a signal relating to a first body movement, a signal relating to a first cardiac activity, a responsiveness signal, an awareness signal, a signal relating to a second cardiac activity, a signal relating to a second body movement, a spectral analysis signal relating to the second cardiac activity, and spectral analysis signal relating to the second body movement; and
determining an occurrence of a partial epileptic seizure based upon a correlation of two features, at least one feature being of each of the at least two body signals, wherein:
   the feature of the first cardiac signal is a value outside an interictal reference value range;
   the feature of the first body movement signal is a body movement associated with a partial seizure;
   the feature of the second cardiac activity signal is a correlation with an ictal cardiac activity reference signal;
   the feature of the second body movement signal is a correlation with an ictal body movement reference signal;
   the feature of the spectral analysis signal of the second cardiac activity is a correlation with an ictal cardiac activity spectral analysis reference signal; or
   the feature of the spectral analysis signal of the second body movement is a correlation with an ictal body movement spectral analysis reference signal; and
performing a further action in response to the determination of the occurrence of the epileptic event.

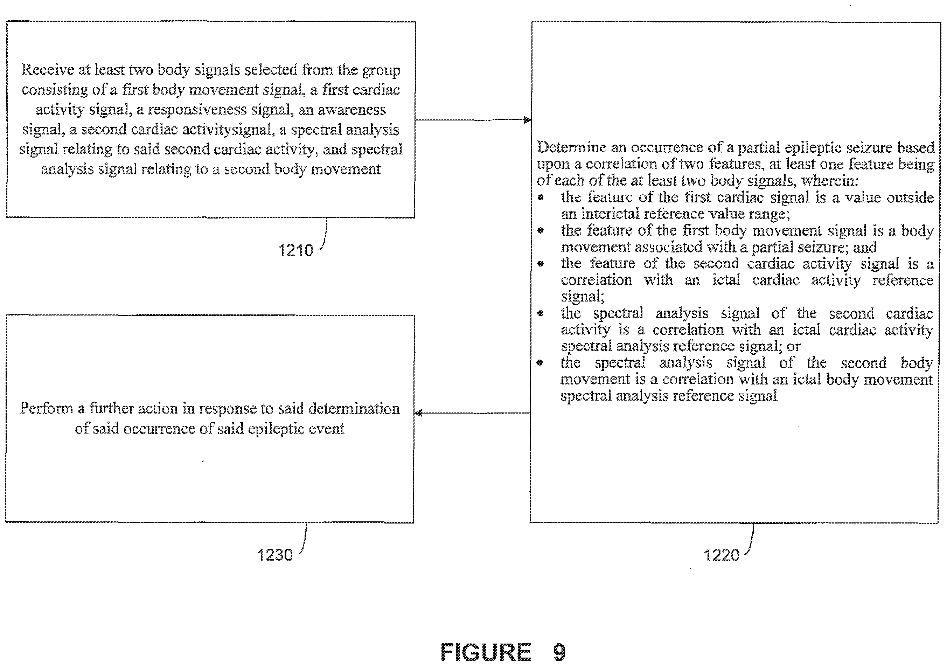

Figure 9 depicts one embodiment of this method. Figure 9 depicts a receiving step 1210, a determining step 1220, and a performing step 1230.

The various signals can be provided by any appropriate technique and their features referred to above can likewise be measured. For example, in one embodiment, the correlation of the second cardiac activity signal with the ictal cardiac activity reference signal comprises a match to an ictal cardiac activity template;
the correlation of the second body movement signal with the ictal body movement reference signal comprises a match to an ictal body movement template;
the correlation of the spectral analysis signal of the second cardiac activity with the ictal cardiac activity spectral analysis reference signal comprises a match to an ictal cardiac activity spectral analysis pattern or template; or
the correlation of the spectral analysis signal of the second body movement with the ictal body movement spectral analysis reference signal comprises a match to an ictal body movement spectral analysis pattern or template.

Matches to patterns and templates are described in U.S. Pat. Appl. 12/884,051, filed September 16, 2010. A "match" should not be construed as requiring a complete or perfect fit to a pattern or template.

In one embodiment, the further action comprises one or more of logging the occurrence and/or time of occurrence of the seizure; providing a warning, alarm or alert to the patient, a caregiver or a health care provider; providing a therapy to prevent, abort, and/or reduce the severity of the seizure; assessing one or more patient parameters such as awareness or responsiveness during the seizure; assessing the severity of the seizure, identifying the end of the seizure; and assessing the patient's post-ictal impairment or recovery from the seizure.

Partial seizures generally result in body movements that do not include falls.

The partial seizure can be classified as
(i) complex if at least one of the features of the awareness signal is a decrease in the patient's awareness below its reference value, or as
(ii) simple if there is no decrease in the patient's awareness below its reference value, or if there is a decrease in the patient's responsiveness but awareness remains at an interictal value.

In one embodiment, the end of the partial epileptic seizure can be indicated when at least one of the features of the respective body signals is outside the range of values associated with the ictal state for that body signal. In another embodiment, the end of the partial epileptic seizure can be indicated when each of the features of the respective body signals trends toward an interictal reference value, range, or pattern of the body signal.

In one embodiment, the method further comprises indicating the beginning of a post-ictal period when at least one of the body signals is outside the range of values associated with the ictal and inter-ictal states for that body signal, wherein:
the post-ictal feature of the cardiac signal is a heart rate outside the range of values associated with the ictal state;
the post-ictal feature of the body movement signal is a change in the patient's movement outside the ictal range of values;
the post-ictal feature of the responsiveness signal is an increase in the patient's responsiveness above an ictal reference value but remaining below an inter-ictal reference value; and
the post-ictal feature of the awareness signal is an increase in the patient's awareness above an ictal reference value but remaining below an inter-ictal reference value.

Partial seizures can be distinguished from generalized seizures. Partial seizures that evolve into secondarily generalized seizures can also be distinguished from primarily generalized seizures. Also, within the class of partial seizures, simple partial seizures can be distinguished from complex partial seizures.

In a further embodiment, this method further comprises classifying the partial epileptic seizure as a complex partial seizure if a feature of the awareness signal timewise correlated with the at least one body signals is a decrease in the patient's awareness or other cognitive functions below an interictal reference value, and as a simple partial seizure if the patient's awareness or other cognitive function remain at or above an inter-ictal reference a feature of the awareness s signal timewise correlated with the at least two body signals.

Alternatively or in addition, in one embodiment, the method further comprises indicating the end of the partial epileptic seizure when at least one of the signal features the respective body signal is outside the range of values for the ictal and interictal periods for that patient and within the range of postictal values;

Alternatively or in addition, in one embodiment, the method further comprises indicating the beginning of a post-ictal period based upon the appearance of at least one post-ictal feature of at least one said body signal, wherein:
the cardiac signal is a heart rate outside an ictal reference value range and within a range indicative of a post-ictal state for said patient;
the accelerometer signal is a movement velocity, amplitude, or number of movements per unit time outside an ictal reference range of values and within a range indicative of a post-ictal state for said patient;
the accelerometer signal is a movement pattern, trajectory, or inter-movement intervals outside an ictal reference value range and within a range indicative of a post-ictal state for said patient;
the respiratory signal is a respiration rate outside the ictal range of values for that patient and within a post-ictal range for said patient;
the responsiveness signal is a change in the patient's unawareness or cognitive dysfunction to a value outside both of an ictal range and an interictal range; and
the awareness signal is a change in the patient's awareness to a value outside both an ictal range and an interictal range.

In a further embodiment, the method further comprises indicating the end of the post-ictal period when each of the features from the respective body signal returns to the range of values associated with the interictal period.

Alternatively or in addition, in one embodiment, the method further comprises indicating the beginning of the inter-ictal period based upon the appearance of at least one inter-ictal feature of at least two said body signal, wherein:
the inter-ictal feature of the cardiac signal is a return of the patient's heart rate values to inter-ictal reference values and outside a range indicative of a post-ictal and ictal state for said patient;
the inter-ictal feature of the accelerometer signal is a return of the patient's movement velocities, amplitudes, or number of movements per unit time to the inter-ictal value range for that patient and outside the values or patterns indicative of a post-ictal and ictal states;
the inter-ictal feature of the accelerometer signal is a return of the movement patterns or trajectories to those present in the inter-ictal period for that patient and different from those present during the post-ictal and ictal states;
the inter-ictal feature of the respiratory signal is return of the respiratory frequency to inter-ictal values for that patient and outside those indicative of post-ictal and ictal states;
the inter-ictal feature of the responsiveness signal is a return of the patient's responsiveness to a range of values seen in the inter-ictal state for that patient and outside a range of values indicative of post-ictal and/or ictal states
the inter-ictal feature of the awareness signal is a return of the patient's awareness to a range of values seen in the inter-ictal state for the patient and outside a range of values indicative of post-ictal and/or ictal states.

Regardless of how an epileptic event is detected, in some embodiments, a responsive action may be taken selected from warning, logging the time of an epileptic event, computing and storing one or more seizure severity indices, or delivering a therapy to prevent, abate or lessen the severity of the ictal or postictal states. Further responsive actions such as warning, logging and treatment may be taken if the ictal or postictal states severity exceeds for example the 90^{th} percentile values for a patient.

A warning may be given as, for example, a warning tone or light implemented by a medical device or a device adapted to receive indications of the seizure; as an automated email, text message, telephone call, or video message sent from a medical device or a unit in communication with a medical device to the patient's cellular telephone, PDA, computer, television, 911 or another emergency contact number for paramedic/EMT services, etc. Such a warning may allow the patient or his or her caregivers to take measures protective of patient's well-being and those of others, *e.g.*, pulling out of traffic and turning off a car, when the patient is driving; stopping the use of machinery, contacting another adult if the patient is providing childcare, removing the patient from a swimming pool or bathtub, lying down or sitting if the patient is standing, etc.

The time may be logged by receiving an indication of the current time and associating the indication of the current time with an indication of the epileptic event.

Various responsive actions, such as warning, logging, and treating, among others, are generally described in U.S. Pat. Appl. No. 12/896,525, filed October 1, 2010. A warning may be graded, e.g., a yellow light for a mild seizure, a red light for a severe one. Treating can comprise providing supporting treatment (e.g., fluids, oxygen). Seizure severity indices may be calculated and stored by appropriate techniques and apparatus. More information on seizure severity indices is available in U.S. Pat. Appl. No. 13/040,996, filed March 4, 2011.

A seizure may be treated by appropriate techniques, such as those discussed below. The treatment may be one or more treatments known in the art. In one embodiment, the treatment comprises at least one of applying an electrical signal to a neural structure of a patient; delivering a drug to a patient; or cooling a neural structure of a patient. When the treatment comprises applying an electrical signal to a portion of a neural structure of a patient, the neural structure may be at least one of a portion of a brain structure of the patient, a portion of a cranial nerve of a patient, a portion of a spinal cord of a patient, a portion of a sympathetic nerve structure of the patient, a portion of a parasympathetic nerve structure of the patient, and/or a portion of a peripheral nerve of the patient.

Though not intended to be bound by theory, in certain circumstances, an epileptic event may be identified at a time before event onset would be determined by electroencephalography, observation by a physician or knowledgeable layman, or both. The time before onset may range from a few seconds up to a few minutes. As such, certain embodiments of the method may be considered to yield a prediction of an epileptic event. It should be noted that the prediction may sometimes be a false positive. However, depending on a physician's judgment, his or her understanding of the devices in use, and the patient's condition, a certain amount of false positives may be tolerable.

Even if no prediction is made, i.e., the methods of various embodiments of this disclosure are capable of identifying an epileptic event at or after the time of electrographic onset, such information may be useful for identifying an epileptic event without the need for EEG monitoring, implanted sensors, or clinical observation, and with a higher signal-to-noise ratio than EEG monitoring using scalp electrodes. Even though scalp recordings are the most common modality for seizure detection, this modality has low sensitivity (e.g., a large number of epileptic seizures are not accompanied by electrical changes at the scalp), low specificity (e.g., muscle and movement artifacts may resemble electrical seizure activity at the scalp), and also may have long latency between the emergence of epileptic activity in certain brain regions and the appearance, if any, of electrical activity at the scalp.

In one embodiment, the present disclosure relates to a system, comprising:
at least one sensor configured to receive at least one of a signal relating to a first cardiac activity from a patient, a signal relating to a first body movement from the patient, a responsiveness signal from the patient, an awareness signal from the patient, a signal relating to a second cardiac activity of the patient, and a signal relating to a second body movement of the patient;
a detection unit configured to receive the at least one signal from the at least one sensor and determine an occurrence of an epileptic event; and
an action unit configured to receive an indication of the occurrence of the epileptic event from the detection unit and perform at least one of logging the occurrence and/or time of occurrence of the epileptic event; providing a warning, alarm or alert to the patient, a caregiver or a health care provider; providing a therapy to prevent, abort, and/or reduce the severity of the epileptic event; assessing one or more patient parameters such as awareness or responsiveness during the epileptic event; assessing the severity of the epileptic event, identifying the end of the epileptic event; and assessing the patient's post-ictal impairment or recovery from the epileptic event.

The system can further comprise other units. For example, the system can comprise a spectral analysis unit configured to generate at least one spectral analysis signal from the signal relating to the second cardiac activity and/or the signal relating to the second body movement. In this embodiment, it may be desirable for the detection unit to be further configured to receive the at least one spectral analysis signal from the spectral analysis unit.

Although not limited to the following, exemplary systems capable of implementing embodiments of the present disclosure are generally discussed below.

Figure 10A depicts a stylized system comprising an external unit 145a capable of receiving, storing, communicating, and/or calculating information relating a patient's epileptic events. The system shown in Figure 10A also includes at least one sensor 212. The sensor 212 may be configured to receive cardiac activity data, body movement data, responsiveness data, awareness data, or other data from the patient's body. A lead 211 is shown allowing communication between the sensor 212 and the external unit 145a.

Figure 10B depicts a stylized implantable medical system (IMD) 100 for implementing one or more embodiments of the present disclosure. An electrical signal generator 110 is provided, having a main body 112 comprising a case or shell with a header 116 for connecting to an insulated, electrically conductive lead assembly 122. The generator 110 is implanted in the patient's chest in a pocket or cavity formed by the implanting surgeon just below the skin (indicated by a dotted line 145), similar to the implantation procedure for a pacemaker pulse generator.

A nerve electrode assembly 125, preferably comprising a plurality of electrodes having at least an electrode pair, is conductively connected to the distal end of the lead assembly 122, which preferably comprises a plurality of lead wires (e.g., one wire for each electrode). Each electrode in the electrode assembly 125 may operate independently or alternatively, may operate in conjunction with the other electrodes. In one embodiment, the electrode assembly 125 comprises at least a cathode and an anode. In another embodiment, the electrode assembly comprises one or more unipolar electrodes.

Lead assembly 122 is attached at its proximal end to connectors on the header 116 of generator 110. The electrode assembly 125 may be surgically coupled to the vagus nerve 127 in the patient's neck or at another location, e.g., near the patient's diaphragm or at the esophagus/stomach junction. Other (or additional) cranial nerves such as the trigeminal and/or glossopharyngeal nerves may also be used to deliver the electrical signal in particular alternative embodiments. In one embodiment, the electrode assembly 125 comprises a bipolar stimulating electrode pair 126, 128 (i.e., a cathode and an anode). Suitable electrode assemblies are available from Cyberonics, Inc., Houston, Texas, USA as the Model 302 electrode assembly. However, persons of skill in the art will appreciate that many electrode designs could be used in the present disclosure. In one embodiment, the two electrodes are wrapped about the vagus nerve, and the electrode assembly 125 may be secured to the vagus nerve 127 by a spiral anchoring tether 130 such as that disclosed in

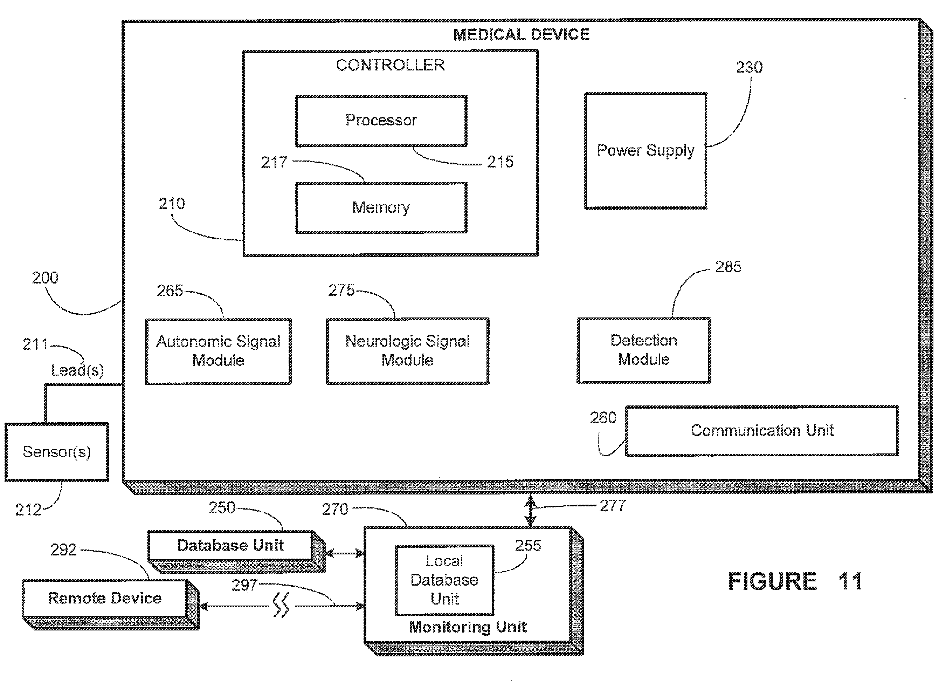

Turning now to Figure 11, a block diagram depiction of a medical device 200 is provided, in accordance with one illustrative embodiment of the present disclosure. In some embodiments, the medical device 200 may be implantable (such as implantable electrical signal generator 110 from Figure 10), while in other embodiments the medical device 200 may be completely external to the body of the patient.

The medical device 200 may comprise a controller 210 capable of controlling various aspects of the operation of the medical device 200. The controller 210 is capable of receiving internal data or external data, and in one embodiment, is capable of causing a stimulation unit (not shown) to generate and deliver an electrical signal, a drug, cooling, or two or more thereof to one or more target tissues of the patient's body for treating a medical condition. For example, the controller 210 may receive manual instructions from an operator externally, or may cause an electrical signal to be generated and delivered based on internal calculations and programming. In other embodiments, the medical device 200 does not comprise a stimulation unit. In either embodiment, the controller 210 is capable of affecting substantially all functions of the medical device 200.

The controller 210 may comprise various components, such as a processor 215, a memory 217, etc. The processor 215 may comprise one or more microcontrollers, microprocessors, etc., capable of performing various executions of software components. The memory 217 may comprise various memory portions where a number of types of data (e.g., internal data, external data instructions, software codes, status data, diagnostic data, etc.) may be stored. The memory 217 may comprise one or more of random access memory (RAM), dynamic random access memory (DRAM), electrically erasable programmable read-only memory (EEPROM), flash memory, etc.

The medical device 200 may also comprise a power supply 230. The power supply 230 may comprise a battery, voltage regulators, capacitors, etc., to provide power for the operation of the medical device 200, including delivering the therapeutic electrical signal. The power supply 230 comprises a power source that in some embodiments may be rechargeable. In other embodiments, a non-rechargeable power source may be used. The power supply 230 provides power for the operation of the medical device 200, including electronic operations and the electrical signal generation and delivery functions. The power supply 230 may comprise a lithium/thionyl chloride cell or a lithium/carbon monofluoride (LiCFx) cell if the medical device 200 is implantable, or may comprise conventional watch or 9V batteries for external (i.e., non-implantable) embodiments. Other battery types known in the art of medical devices may also be used.

The medical device 200 may also comprise a communication unit 260 capable of facilitating communications between the medical device 200 and various devices. In particular, the communication unit 260 is capable of providing transmission and reception of electronic signals to and from a monitoring unit 270, such as a handheld computer or PDA that can communicate with the medical device 200 wirelessly or by cable. The communication unit 260 may include hardware, software, firmware, or any combination thereof.

The medical device 200 may also comprise one or more sensor(s) 212 coupled via sensor lead(s) 211 to the medical device 200. The sensor(s) 212 are capable of receiving signals related to a physiological parameter, such as the patient's heart beat, blood pressure, and/or temperature, and delivering the signals to the medical device 200. The sensor 212 may also be capable of detecting kinetic signal associated with a patient's movement. The sensor 212, in one embodiment, may be an accelerometer. The sensor 212, in another embodiment, may be an inclinometer. In another embodiment, the sensor 212 may be an actigraph. In one embodiment, the sensor(s) 212 may be the same as implanted electrode(s) 126, 128 (Figure 10). In other embodiments, the sensor(s) 212 are external structures that may be placed on the patient's skin, such as over the patient's heart or elsewhere on the patient's torso. The sensor 212, in one embodiment is a multimodal signal sensor capable of detecting various autonomic and neurologic signals, including kinetic signals associated with the patient's movement.

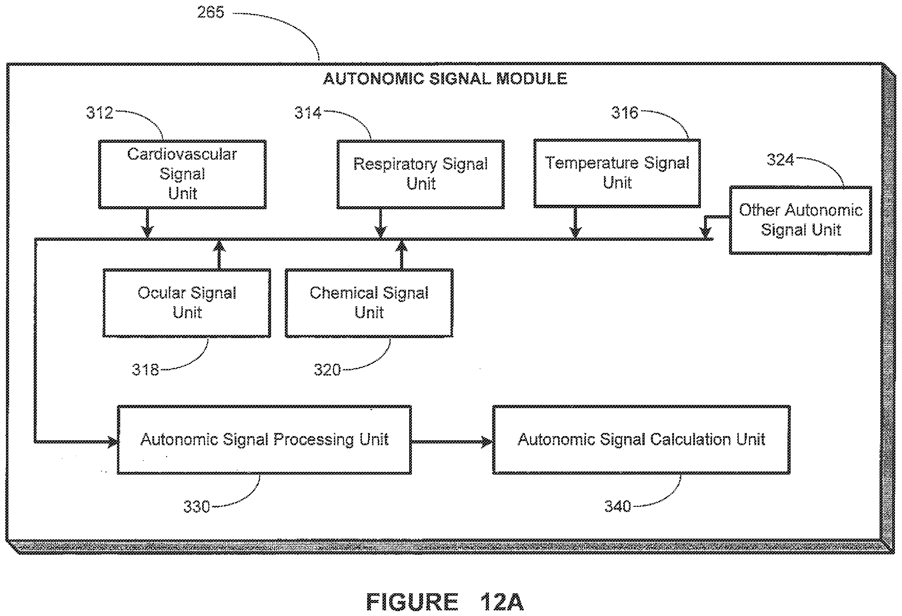

In one embodiment, the medical device 200 may comprise a autonomic signal module 265 that is capable of collecting autonomic data, e.g., cardiac data comprising fiducial time markers of each of a plurality of heart beats. The autonomic signal module 265 may also process or condition the autonomic data. The autonomic data may be provided by the sensor(s) 212. The autonomic signal module 265 may be capable of performing any necessary or suitable amplifying, filtering, and performing analog-to-digital (A/D) conversions to prepare the signals for downstream processing. The autonomic data module 265, in one embodiment, may comprise software module(s) that are capable of performing various interface functions, filtering functions, etc.. In another embodiment, the autonomic signal module 265 may comprise hardware circuitry that is capable of performing these functions. In yet another embodiment, the autonomic signal module 265 may comprise hardware, firmware, software and/or any combination thereof. A more detailed illustration of the autonomic signal module 265 is provided in Figure 12A and accompanying description below.

The autonomic signal module 265 is capable of collecting autonomic data and providing the collected autonomic data to a detection module 285.

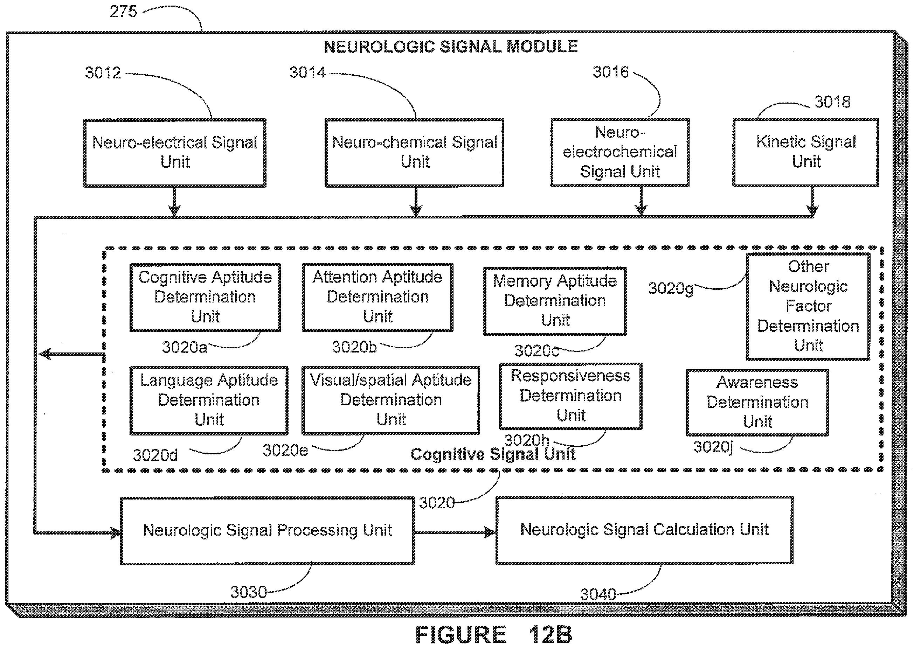

In one embodiment, the medical device 200 may comprise a neurological signal module 275 that is capable of collecting neurologic data, e.g., kinetic signals indicative of the patient's movement. The neurological signal module 275 may also process or condition the neurologic data. The neurologic data may be provided by the sensor(s) 212. The neurological signal module 275 may be capable of performing any necessary or suitable amplifying, filtering, and performing analog-to-digital (A/D) conversions to prepare the signals for downstream processing. The neurological signal module 275, in one embodiment, may comprise software module(s) that are capable of performing various interface functions, filtering functions, etc.. In another embodiment, the neurological signal module 275 may comprise hardware circuitry that is capable of performing these functions. In yet another embodiment, the neurological signal module 275 may comprise hardware, firmware, software and/or any combination thereof. Further description of the neurologic signal module 275 is provided in Figure 12B and accompanying description below.

The neurological signal module 275 is capable of collecting autonomic data and providing the collected autonomic data to a detection module 285.

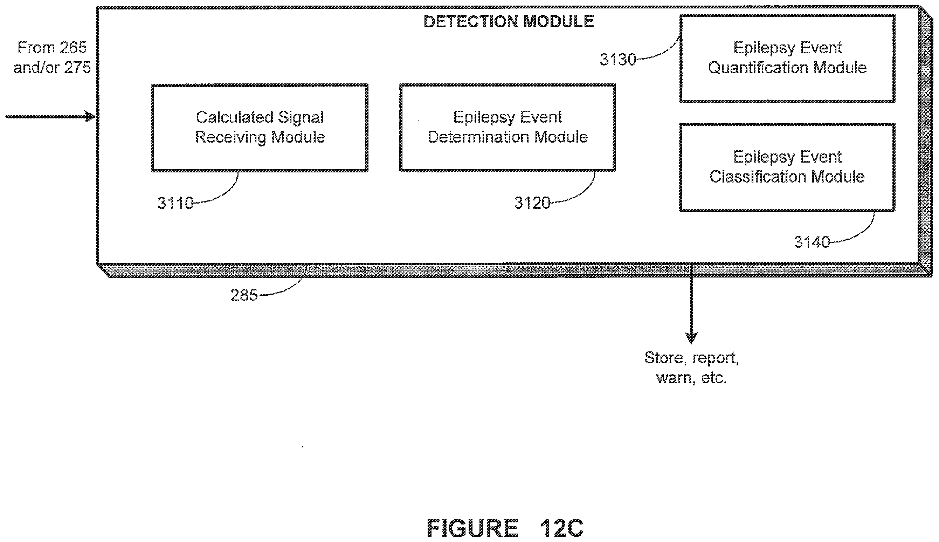

The detection module 285 is capable of detecting an epileptic event based upon an autonomic signal provided by autonomic signal module 265 and neurological signal module 275. The detection module 285 can implement one or more algorithms using the autonomic data and neurologic data in any particular order, weighting, etc. The detection module 285 may comprise software module(s) that are capable of performing various interface functions, filtering functions, etc. In another embodiment, the detection module 285 may comprise hardware circuitry that is capable of performing these functions. In yet another embodiment, the detection module 285 may comprise hardware, firmware, software and/or any combination thereof. Further description of the detection module 285 is provided in Figure 12C and accompanying description below.

In addition to components of the medical device 200 described above, a medical device system may comprise a storage unit to store an indication of at least one of seizure or an increased risk of a seizure. The storage unit may be the memory 217 of the medical device 200, another storage unit of the medical device 200, or an external database, such as a local database unit 255 or a remote database unit 250. The medical device 200 may communicate the indication via the communications unit 260. Alternatively or in addition to an external database, the medical device 200 may be adapted to communicate the indication to at least one of a patient, a caregiver, or a healthcare provider.

In various embodiments, one or more of the units or modules described above may be located in a monitoring unit 270 or a remote device 292, with communications between that unit or module and a unit or module located in the medical device 200 taking place via communication unit 260. For example, in one embodiment, one or more of the autonomic signal module 265, the neurologic signal module 275, or the detection module 285 may be external to the medical device 200, e.g., in a monitoring unit 270. Locating one or more of the autonomic signal module 265, the neurologic signal module 275, or the detection module 285 outside the medical device 200 may be advantageous if the calculation(s) is/are computationally intensive, in order to reduce energy expenditure and heat generation in the medical device 200 or to expedite calculation.

The monitoring unit 270 may be a device that is capable of transmitting and receiving data to and from the medical device 200. In one embodiment, the monitoring unit 270 is a computer system capable of executing a data-acquisition program. The monitoring unit 270 may be controlled by a healthcare provider, such as a physician, at a base station in, for example, a doctor's office. In alternative embodiments, the monitoring unit 270 may be controlled by a patient in a system providing less interactive communication with the medical device 200 than another monitoring unit 270 controlled by a healthcare provider. Whether controlled by the patient or by a healthcare provider, the monitoring unit 270 may be a computer, preferably a handheld computer or PDA, but may alternatively comprise any other device that is capable of electronic communications and programming, e.g., hand-held computer system, a PC computer system, a laptop computer system, a server, a personal digital assistant (PDA), an Apple-based computer system, a cellular telephone, etc. The monitoring unit 270 may download various parameters and program software into the medical device 200 for programming the operation of the medical device, and may also receive and upload various status conditions and other data from the medical device 200. Communications between the monitoring unit 270 and the communication unit 260 in the medical device 200 may occur via a wireless or other type of communication, represented generally by line 277 in Figure 11. This may occur using, *e.g.*, wand 155 (Figure 10) to communicate by RF energy with an implantable signal generator 110. Alternatively, the wand may be omitted in some systems, e.g., systems in which the MD 200 is non-implantable, or implantable systems in which monitoring unit 270 and MD 200 operate in the MICS bandwidths.

In one embodiment, the monitoring unit 270 may comprise a local database unit 255. Optionally or alternatively, the monitoring unit 270 may also be coupled to a database unit 250, which may be separate from monitoring unit 270 (e.g., a centralized database wirelessly linked to a handheld monitoring unit 270). The database unit 250 and/or the local database unit 255 are capable of storing various patient data. These data may comprise patient parameter data acquired from a patient's body, therapy parameter data, seizure severity data, and/or therapeutic efficacy data. The database unit 250 and/or the local database unit 255 may comprise data for a plurality of patients, and may be organized and stored in a variety of manners, such as in date format, severity of disease format, etc. The database unit 250 and/or the local database unit 255 may be relational databases in one embodiment. A physician may perform various patient management functions (*e.g.*, programming parameters for a responsive therapy and/or setting references for one or more detection parameters) using the monitoring unit 270, which may include obtaining and/or analyzing data from the medical device 200 and/or data from the database unit 250 and/or the local database unit 255. The database unit 250 and/or the local database unit 255 may store various patient data.

One or more of the blocks illustrated in the block diagram of the medical device 200 in Figure 11 may comprise hardware units, software units, firmware units, or any combination thereof. Additionally, one or more blocks illustrated in Figure 11 may be combined with other blocks, which may represent circuit hardware units, software algorithms, etc. Additionally, any number of the circuitry or software units associated with the various blocks illustrated in Figure 11 may be combined into a programmable device, such as a field programmable gate array, an ASIC device, etc.

Turning to Figure 12A, an autonomic signal module 265 is shown in more detail. The autonomic signal module 265 can comprise a cardiovascular signal unit 312 capable of providing at least one cardiovascular signal. Alternatively or in addition, the autonomic signal module 265 can comprise a respiratory signal unit 314 capable of providing at least one respiratory signal. Alternatively or in addition, the autonomic signal module 265 can comprise a blood parameter signal unit 323capable of providing at least one blood parameter signal (e.g., blood glucose, blood pH, blood gas, etc.).Alternatively or in addition, the autonomic signal module 265 can comprise a temperature signal unit 316 capable of providing at least one temperature signal. Alternatively or in addition, the autonomic signal module 265 can comprise an optic signal unit 318 capable of providing at least one optic signal. Alternatively or in addition, the autonomic signal module 265 can comprise a chemical signal unit 320 capable of providing at least one body chemical signal. Alternatively or in addition, the autonomic signal module 265can comprise a hormone signal unit 322 capable of providing at least one hormone signal. Alternatively or in addition, the autonomic signal module 265 can comprise one or more other autonomic signal unit(s) 324, such as a skin resistance signal unit.

The autonomic signal module 265 can also comprise an autonomic signal processing unit 330. The autonomic signal processing unit 330 can perform any filtering, noise reduction, amplification, or other appropriate processing of the data received by the signal units 312-324 desired by the person of ordinary skill in the art prior to calculation of the autonomic signal.

The autonomic signal module 265 can also comprise an autonomic signal calculation unit 340. The autonomic signal calculation unit 340 can calculate an autonomic signal from the data passed by the autonomic signal processing unit 330. A calculated autonomic signal herein refers to any signal derivable from the provided signals, with or without processing by the autonomic signal processing unit 330.

For example, the autonomic signal calculation unit 340 may calculate the heart rate, a change in the heart rate, the speed of change in heart rate, blood pressure, heart sounds, heart rhythm, heartbeat morphology at various scales (see, *e.g.*, US 12/884,051, filed September 16, 2010, and US 12/886,419, filed September 20, 2010, which are hereby incorporated herein by reference), or the shape of the deflection of the thoracic wall as the heart apex beats against it, among others, from cardiovascular data received by cardiovascular signal unit 312.

For another example, the autonomic signal calculation unit 340 may calculate the respiration (breath) rate, respiration pattern, airflow velocity, respiration amplitude (tidal volume, minute volume), arterial gas concentrations such as end-tidal carbon dioxide, among others, from respiratory data received by respiratory signal unit 314.

For still another example, the autonomic signal calculation unit 340 may calculate a change in the skin temperature or skin electrical resistance of a part of the patient's face or a change in the core temperature of the patient, from temperature data received by temperature signal unit 316.

Turning to Figure 12B, an exemplary embodiment of a neurologic signal module 275 is shown. The neurologic signal module 275 can comprise at least one of a neuro-electrical signal unit 3012 capable of providing at least one neuro-electrical signal; a neuro-chemical signal unit 3014 capable of providing at least one neuro-chemical signal; a neuro-electrochemical signal unit 3016 capable of providing at least one neuro-electrochemical signal; a kinetic signal unit 3018 capable of providing at least one kinetic signal; or a cognitive signal unit 3020 capable of providing at least one cognitive signal. The cognitive signal unit 3020 may be a component of a remote device.

In one embodiment, the cognitive signal unit comprises at least one of a cognitive aptitude determination unit 3020a capable of processing at least one cognitive aptitude indication; an attention aptitude determination unit 3020b capable of processing at least one attention aptitude indication; a memory aptitude determination unit 3020c capable of processing at least one memory indication; a language aptitude determination unit 3020d capable of processing at least one language indication; a visual/spatial aptitude determination unit 3020e capable of processing at least one visual/spatial indication; one or more other neurologic factor determination unit(s) 3020g; a responsiveness determination unit 3020h; or an awareness determination unit 3020j.

The neurologic signal module 275 can also comprise a neurologic signal processing unit 3030. The neurologic signal processing unit 3030 can perform any filtering, noise reduction, amplification, or other appropriate processing of the data received by the signal units 3012-3020 desired by the person of ordinary skill in the art prior to calculation of the neurologic signal.

The neurologic signal module 275 can also comprise a neurologic signal calculation unit 3040. The neurologic signal calculation unit 3040 can calculate a neurologic signal from the data passed by the neurologic signal processing unit 3030. A calculated neurologic signal herein refers to any signal derivable from the provided signals.

For example, the neurologic signal calculation unit 3040 may calculate a brain activity, such as those determinable from signals yielded by an EEG, ECoG, or depth electrodes (i.e., a deep brain electrode), as received by neuro-electrical signal unit 3012, neuro-chemical signal unit 3014, and/or neuro-electrochemical signal unit 3016 and, optionally, further processed by neurologic data processing unit 3030.

A calculated signal regarding brain activity can also be calculated using other neurological signals. For example, spikes in neurons or axons in the brain and spinal cord including central structures and pathways with autonomic control or modulatory capabilities, cranial nerves (e.g., vagus nerve), autonomic ganglia or nerves and peripheral nerves can be sensed and signals provided. Neural imaging or brain imaging signals may be provided, including, for example: Functional Magnetic Resonance Imaging (fMRI), Magnetoencephalography (MEG), Positron Emission Tomography (PET), Event-Related Optical Signal (EROS), and Diffuse Optical Imaging (DOI)). Other imaging techniques such as voltage-sensitive dyes, ultrasound, infra-red, near infra-red and other forms of thermography may also provide signals from which a brain activity can be calculated.

For another example, the neurologic signal calculation unit 3040 may calculate a body kinetic signal, such as the body's (or of a portion thereof such as the head, an arm, or a leg) acceleration; direction; position; smoothness, amplitude, force of movements and number of movements per unit time, and whether there are extraneous or abnormal body oscillations during resting conditions or movement. The signal may be provided by electromyography, a mechanogram, an accelerometer, an actigraph, and/or an inclinometer, as received by kinetic capability determination unit 3018, and, optionally, further processed by neurologic data processing unit 3030.

Kinetic signals provide insight into the functional state of the nervous system and are thus classified as a neurologic signal. The ability to perform movements: a) in any direction; b) do it smoothly and with precision so that for example, a target (e.g. putting a key into its hole) may be met in the first attempt or handwriting is legible; c) changing direction to avoid colliding with an object interposed on its path to a target and re-adjusting the trajectory to reach the original target; and d) with adaptive force and discriminations so to be able to pick a penny off a flat surface and also lift heavy objects. The acceleration and velocity speed, direction and smoothness may be quantified using tools such as 3-D accelerometers among others.

Turning to Figure 12C, a block diagram of detection module 285 is depicted. The detection module 285 comprises a calculated signal receiving module 3110 capable of receiving data indicative of a calculated signal from one or more of the autonomic signal module 265 and the neurologic signal module 275 or a memory 217 storing prior outputs of such a module, and epilepsy event determination module 3120 capable of determining from the received data the occurrence of an epileptic event, e.g., a seizure. The epilepsy event determination module 3120 may implement any appropriate algorithms for determining an epilepsy event from autonomic signals and neurologic signals, e.g., from cardiac data and kinetic data, such as those referred to above.

In the embodiment shown in Figure 12C, the detection module 285 further comprises an epilepsy event quantification module 3130 capable of quantifying from the received data one or more quantifiable characteristics of an epileptic event, e.g., a seizure. Exemplary quantifiable characteristics include, but are not limited to, event duration, duration of stages of the event (*e.g.*, preictal, ictal, and/or postictal stages), values and/or ranges thereof of one or more body signals (*e.g.*, a peak heart rate, a time series of heart rate, etc.), among others.

In the embodiment shown in Figure 12C, the detection module 285 also comprises an epilepsy event classification module 3140 capable of classifying an epileptic event, e.g., a seizure, *e.g.*, as a partial seizure, a generalized seizure, or an absence seizure; as a simple partial or complex partial seizure; as a primarily generalized seizure or a secondarily generalized seizure, etc. This module may be also used to classify events as epileptic or non-epileptic (e.g., pseudo-seizures, psychogenic seizures, etc.)Although Figure 12C shows both an epilepsy event quantification module 3130 and an epilepsy event classification module 3140, in other embodiments, either or both of modules 3130-3140 may be omitted.

The detection module 285 may send the output of the epilepsy event determination module 3120 to one or more other modules of the medical device 200 and/or one or more external units. The one or more other modules may then store the output, report the output to the patient, a physician, and/or a caregiver; warn the patient or a caregiver of an epileptic event, etc.

The medical device system of one embodiment of the present disclosure provides for software module(s) that are capable of acquiring, storing, and processing various forms of data, such as patient data/parameters (e.g., physiological data, side-effects data, heart rate data, breathing rate data, brain-activity parameters, disease progression or regression data, quality of life data, etc.) and therapy parameter data. Therapy parameters may include, but are not limited to, electrical signal parameters (e.g., frequency, pulse width, wave shape, polarity, geometry of electrical fields, on-time, off-time, etc.) that define therapeutic electrical signals delivered by the medical device in response to the detection of the seizure, medication type, dose, or other parameters, and/or any other therapeutic treatment parameter.

In one embodiment, the medical device 200 or an external unit 270 may also be capable of detecting a manual input from the patient. The manual input may include a magnetic signal input, a tap input, a button, dial, or switch input, a touchscreen input, a wireless data input to the medical device 200, etc. The manual input may be used to allow capture of the patient's subjective assessment of his or her epileptic events. For example, the medical device 200 may comprise one or more physical or virtual (e.g., touchscreen-implemented) buttons accessible to the patient's fingers or a caregiver's, through which the patient or caregiver may indicate he or she is having an epileptic event or is not having an epileptic event. Alternatively or in addition, the manual input may be used to gauge the patient's responsiveness.

The above methods may be performed by a computer readable program storage device encoded with instructions that, when executed by a computer, perform the method described herein.

All of the methods and apparatuses disclosed and claimed herein may be made and executed without undue experimentation in light of the present disclosure. While the methods and apparatus of this disclosure have been described in terms of particular embodiments, it will be apparent to those skilled in the art that variations may be applied to the methods and apparatus and in the steps, or in the sequence of steps, of the method described herein without departing from the appended claims. It should be especially apparent that the principles of the disclosure may be applied to selected cranial nerves other than, or in addition to, the vagus nerve to achieve particular results in treating patients having epilepsy, depression, or other medical conditions.

## Claims

1. A medical device system for detecting an epileptic seizure based upon a patient's cardiac signal and body movement, comprising:
a heart beat sensor for sensing a cardiac signal indicative of the patient's heart beats; a motion sensor for sensing a kinetic signal indicative of a body movement of the patient;
a detection module for detecting an epileptic seizure based upon the cardiac signal and the kinetic signal;
wherein the detection module comprises a program storage device having an algorithm for detecting an epileptic seizure, said algorithm when executed being capable of performing a method comprising the steps of detecting a possible epileptic seizure based upon changes in the patient's cardiac signal;
calculating at least one kinetic score for said kinetic signal, said at least one kinetic score being indicative of a correlation of said kinetic signal with an epileptic seizure;
confirming the detection of the possible epileptic seizure if the kinetic score is indicative of a high correlation with an epileptic seizure; and
overriding the detection of the possible epileptic seizure if the kinetic score is indicative of a low correlation with an epileptic seizure; and
providing an output indicative of the epileptic seizure if the detecting is confirmed; and
further comprising an epileptic event classification module for classifying said epileptic seizure as a tonic-clonic, simple partial or complex partial seizure based upon at least one of said cardiac signal and said kinetic signal.

2. The medical device system of claim 1, further comprising
an autonomic signal module coupled to said heart beat sensor for providing an electrocardiogram (EKG) signal.

3. The medical device system of claim 1, wherein the motion sensor comprises one or more of an accelerometer, an inclinometer, and an actigraph.

4. The medical device system of claim 1, further comprising at least one of a responsiveness determination unit for providing a signal indicative of the patient's responsiveness following detecting a seizure, and an epilepsy event quantification module for characterizing the epileptic seizure based upon the responsiveness signal.

5. The medical device system of claim 1, further comprising an awareness determination unit for providing an awareness signal indicative of the patient's awareness following said detecting, and characterizing the epileptic event based upon the awareness signal.

6. The medical device system of claim 1, wherein the detection module comprises a program storage device having an algorithm for detecting the epileptic seizure based upon the temporal relationship between the cardiac signal and the kinetic signal.

7. A medical device system for detecting an epileptic seizure based upon a patient's cardiac signal and body movement, comprising:
a heart beat sensor for sensing a cardiac signal indicative of the patient's heart beats; a motion sensor for sensing a kinetic signal indicative of a body movement of the patient;
a detection module for detecting an epileptic seizure based upon the cardiac signal and the kinetic signal;
wherein the detection module comprises a program storage device having an algorithm for detecting an epileptic seizure, said algorithm when executed being capable of performing a method comprising the steps of
calculating based on the cardiac signal a cardiac score indicative of a correlation of said cardiac signal with an epileptic event; detecting an epileptic event based upon the patient's kinetic activity; and providing an output indicative of an epileptic event based on the cardiac score; and
further comprising an epileptic event classification module for classifying said epileptic seizure as a tonic-clonic, simple partial or complex partial seizure based upon at least one of said cardiac signal and said kinetic signal.

## Patentansprüche

1. Ein medizinisches Vorrichtungssystem zum Detektieren eines epileptischen Anfalls auf der Grundlage eines kardialen Signals und einer Körperbewegung eines Patienten, das umfasst:
einen Herzschlagsensor zum Detektieren eines kardialen Signals, das den Herzschlag des Patienten anzeigt;
einen Bewegungssensor zum Detektieren eines kinetischen Signals, das eine Körperbewegung des Patienten anzeigt;
ein Detektionsmodul zum Detektieren eines epileptischen Anfalls auf der Grundlage des kardialen Signals und des kinetischen Signals;
wobei das Detektionsmodul eine Programmspeichereinrichtung mit einem Algorithmus zum Detektieren eines epileptischen Anfalls umfasst, wobei der genannte Algorithmus, wenn er ausgeführt wird, dazu ausgebildet ist, ein Verfahren auszuführen, das die Schritte umfasst:
Detektieren eines möglichen epileptischen Anfalls auf der Grundlage von Änderungen in dem kardialen Signal des Patienten;
Berechnen von zumindest einer kinetischen Bewertung für das genannte kinetische Signal, wobei die genannte zumindest eine kinetische Bewertung eine Korrelation des genannten kinetischen Signals mit einem epileptischen Anfall anzeigt;
Bestätigen der Detektion des möglichen epileptischen Anfalls, wenn die kinetische Bewertung eine hohe Korrelation mit einem epileptischen Anfall anzeigt; und
Außerkraftsetzen der Detektion des möglichen epileptischen Anfalls, wenn die kinetische Bewertung eine niedrige Korrelation mit einem epileptischen Anfall anzeigt; und
Ausgeben einer Ausgabe, die den epileptischen Anfall anzeigt, wenn die Detektion bestätigt wird; und
weiterhin mit einem Klassifikationsmodul für ein epileptisches Ereignis zum Klassifizieren des genannten epileptischen Anfalls als einen tonisch-klonischen, einfachen teilweisen oder komplexen teilweisen Anfall auf der Grundlage von zumindest einem von dem genannten kardialen und kinetischen Signal.

2. Das medizinische Vorrichtungssystem von Anspruch 1, das weiterhin umfasst:
ein Autonomes-Signal-Modul, das mit dem genannten Herzschlagsensor verbunden ist; zum Liefern eines Elektrokardiogramm-(EKG-)Signals

3. Das medizinische Vorrichtungssystem von Anspruch 1, in dem der Bewegungssensor eines oder mehreres von einem Beschleunigungsmesser, einem Neigungsmesser und einem Aktigraphen umfasst.

4. Das medizinische Vorrichtungssystem von Anspruch 1, weiterhin mit zumindest einem von einer Reaktionsfähigkeitsbestimmungseinheit zum Liefern eines Signals, das die auf das Detektieren eines Anfalls folgende Reaktionsfähigkeit des Patienten anzeigt, und einem Epilepsieereignisquantifikationsmodul zum Charakterisieren des epileptischen Anfalls auf der Grundlage des Reaktionsfähigkeitssignals.

5. Das medizinische Vorrichtungssystem von Anspruch 1, das weiterhin eine Bewusstheitsbestimmungseinheit zum Liefern eines Bewusstheitssignals, das die auf das genannte Detekieren folgende Bewusstheit des Patienten anzeigt, und Charakterisieren des epileptischen Anfalls auf der Grundlage des Bewusstheitssignals umfasst.

6. Das medizinische Vorrichtungssystem von Anspruch 1, in dem das Detektionsmodul eine Programmspeichereinrichtung mit einem Algorithmus zum Detektieren des epileptischen Anfalls auf der Grundlage des zeitlichen Verhältnisses zwischen dem kardialen Signal und dem kinetischen Signal umfasst.

7. Ein medizinisches Vorrichtungssystem zum Detektieren eines epileptischen Anfalls auf der Grundlage eines kardialen Signals und einer Körperbewegung eines Patienten, das umfasst:
einen Herzschlagsensor zum Detektieren eines kardialen Signals, das den Herzschlag des Patienten anzeigt;
einen Bewegungssensor zum Detektieren eines kinetischen Signals, das eine Körperbewegung des Patienten anzeigt;
ein Detektionsmodul zum Detektieren eines epileptischen Anfalls auf der Grundlage des kardialen Signals und des kinetischen Signals;
wobei das Detektionsmodul eine Programmspeichereinrichtung mit einem Algorithmus zum Detektieren eines epileptischen Anfalls umfasst, wobei der genannte Algorithmus, wenn er ausgeführt wird, dazu ausgebildet ist, ein Verfahren auszuführen, das die Schritte umfasst:
Berechnen einer kardialen Bewertung, die eine Korrelation des genannten kardialen Signals mit einem epileptischen Anfall anzeigt, auf der Grundlage des kardialen Signals;
Detektieren eines epileptischen Ereignisses auf der Grundlage einer kinetischen Aktivität des Patienten; und Ausgeben einer Ausgabe, die ein epileptisches Ereignis anzeigt, auf der Grundlage der kardialen Bewertung; und
weiterhin mit einem Klassifikationsmodul für ein epileptisches Ereignis zum Klassifizieren des genannten epileptischen Anfalls als einen tonisch-klonischen, einfachen teilweisen oder komplex teilweisen Anfall auf der Grundlage von zumindest einem von dem genannten kardialen und kinetischen Signal.

## Revendications

1. Système dispositif médical de détection d'une crise d'épilepsie basée sur un signal cardiaque et un mouvement corporel d'un patient, comprenant :
un capteur de battement du coeur pour capter un signal cardiaque indicatif des battements du coeur du patient ;
un capteur de mouvement pour capter un signal cinétique indicatif d'un mouvement corporel du patient ;
un module de détection pour détecter une crise d'épilepsie sur la base du signal cardiaque et du signal cinétique ;
le module de détection comprenant un dispositif de stockage de programme ayant un algorithme de détection d'une crise d'épilepsie, ledit algorithme lorsqu'il est exécuté étant capable de réaliser un procédé comprenant les étapes de détection d'une crise d'épilepsie possible sur la base des changements dans le signal cardiaque du patient ;
le calcul au moins d'une note cinétique pour ledit signal cinétique, ladite au moins une note cinétique étant indicatrice d'une corrélation dudit signal cinétique avec une crise d'épilepsie ;
la confirmation de la détection de la crise d'épilepsie possible si la note cinétique est indicatrice d'une corrélation élevée avec une crise d'épilepsie ; et
l'outrepassement de la détection de la crise d'épilepsie possible si la note cinétique est indicatrice d'une faible corrélation avec une crise d'épilepsie ; et
la fourniture d'une sortie indicatrice de la crise d'épilepsie si la détection est confirmée ; et
comprenant en outre un module de classification d'évènements d'épilepsie pour classifier ladite crise d'épilepsie comme tonique-clonique, crise partielle simple ou crise partielle complexe sur la base d'au moins l'un dudit signal cardiaque et dudit signal cinétique.

2. Système dispositif médical selon la revendication 1, comprenant en outre
un module signal autonome accouplé audit capteur de battement du coeur pour fournir un signal d'électrocardiogramme (ECG).

3. Système dispositif médical selon la revendication 1, le capteur de mouvement comprenant l'un ou plusieurs parmi un accéléromètre, un inclinomètre, et un actigraphe.

4. Système dispositif médical selon la revendication 1, comprenant en outre au moins l'une d'une unité de détermination de réaction pour fournir un signal indicatif de la réaction du patient suite à la détection d'une crise, et un module de quantification d'évènement d'épilepsie pour caractériser la crise d'épilepsie sur la base du signal de réaction.

5. Système dispositif médical selon la revendication 1, comprenant en outre une unité de détermination de vigilance pour fournir un signal d'avertissement indicatif de la prise de conscience du patient suite à ladite détection, et caractérisant l'évènement d'épilepsie sur la base du signal d'avertissement.

6. Système dispositif médical selon la revendication 1, le module de détection comprenant un dispositif de stockage de programme ayant un algorithme de détection de la crise d'épilepsie sur la base de la relation temporelle entre le signal cardiaque et le signal cinétique.

7. Système dispositif médical de détection d'une crise d'épilepsie basé sur un signal cardiaque et un mouvement corporel d'un patient, comprenant :
un capteur de battement du coeur pour capter un signal cardiaque indicatif des battements du coeur du patient ; un capteur de mouvement pour capter un signal cinétique indicatif d'un mouvement corporel du patient ;
un module de détection pour détecter une crise d'épilepsie sur la base du signal cardiaque et du signal cinétique ;
le module de détection comprenant un dispositif de stockage de programme ayant un algorithme de détection d'une crise d'épilepsie, ledit algorithme lorsqu'il est exécuté étant capable de réaliser un procédé comprenant les étapes de
calcul sur la base du signal cardiaque d'une note cardiaque indicatrice d'une corrélation dudit signal cardiaque avec un événement d'épilepsie ; détection d'un évènement d'épilepsie sur la base de l'activité cinétique du patient ; et fourniture d'une sortie indiquant un événement d'épilepsie basé sur la note cardiaque ; et
comprenant en outre un module de classification d'évènements d'épilepsie pour classifier ladite crise d'épilepsie comme tonique-clonique, crise partielle simple ou crise partielle complexe sur la base d'au moins l'un dudit signal cardiaque et dudit signal cinétique.
